(19) Europäisches Patentamt
European Patent Office
Office européen des brevets

(11) Veröffentlichungsnummer: **0 286 595 B1**

(12) **EUROPÄISCHE PATENTSCHRIFT**

(45) Veröffentlichungstag der Patentschrift: **11.08.93**

(51) Int. Cl.$^5$: **C07C 323/64**, C07D 219/02, C07D 279/24, C07D 241/48, C10M 135/22, C08K 5/36, C09K 15/28

(21) Anmeldenummer: **88810213.4**

(22) Anmeldetag: **30.03.88**

(54) **Schwefelhaltige Verbindungen als Antioxidantien für Schmiermittel und Elastomere.**

(30) Priorität: **08.04.87 CH 1351/87**

(43) Veröffentlichungstag der Anmeldung:
**12.10.88 Patentblatt 88/41**

(45) Bekanntmachung des Hinweises auf die Patenterteilung:
**11.08.93 Patentblatt 93/32**

(84) Benannte Vertragsstaaten:
**AT BE DE ES FR GB IT NL SE**

(56) Entgegenhaltungen:
**EP-A- 0 131 221**
**US-A- 3 344 068**
**US-A- 3 803 140**

**CHEMICAL ABSTRACTS, Band 106, Nr. 23, 8. Juni 1987, Seite 701, Zusammenfassung 196002b, Columbus, Ohio, USA; D. N. TAI et al: "Azomethine and its conversion products IX - Mechanism of the reaction between thioglycolic acid and anomethine base"**

(73) Patentinhaber: **CIBA-GEIGY AG**
**Klybeckstrasse 141**
**CH-4002 Basel(CH)**

(72) Erfinder: **Evans, Samuel, Dr.**
**Route des Charbonnières 17**
**CH-1723 Marly(CH)**

EP 0 286 595 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue schwefelhaltige Verbindungen, ein Verfahren zu ihrer Herstellung und ihre Verwendung zur Stabilisierung von Schmiermitteln, Hydraulikflüssigkeiten und Elastomeren.

Die Stabilisierung von Schmiermitteln und Elastomeren mit Antioxidantien, wie sterisch gehinderten Phenolen, p-Phenylendiaminen oder Diphenylaminderivaten, ist schon lange bekannt. Auch am Stickstoffatom substituierte Diphenylamine oder Phenothiazine sind als Antioxidantien bekannt und z.B. in der DE-OS 2228350 oder in der EP-Patentanmeldung Nr. 70436 beschrieben.

Es wurde nun gefunden, dass sich Aminderivate enthaltend einen am Stickstoffatom gebundenen Thioglykolsäure-, Thiopropionsäure- oder Thioäthyloxyrest zur Stabilisierung von Schmiermitteln und Hydraulikflüssigkeiten, sowie von Elastomeren, besonders gut eignen und hervorragende Antioxidans-Eigenschaften aufweisen.

Die vorliegende Erfindung betrifft demnach Verbindungen der Formel (I)

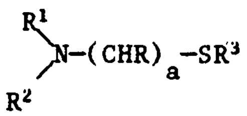

(I),

worin

R unabhängig voneinander -H, Phenyl, Naphthyl oder $C_7$-$C_{30}$-Alkaryl, und

$R^1$ -H, $C_1$-$C_8$-Alkyl, $C_7$-$C_9$-Aralkyl, $C_5$-$C_{12}$-Cycloalkyl, Phenyl, Naphthyl, $C_7$-$C_{30}$-Alkaryl oder eine Gruppe der Formel (II)

$-(CH_2)_a-SR^3$ (II) bedeuten,

$R^2$ $C_7$-$C_{30}$-Alkaryl, Phenyl, Naphthyl oder Phenyl bedeutet, das in para-Stellung eine HO- oder $C_1$-$C_{18}$-Alkoxygruppe oder eine Gruppe der Formeln

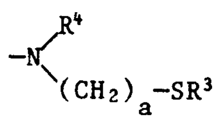

oder

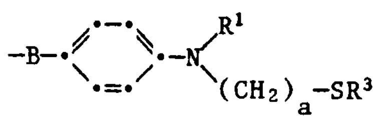

oder

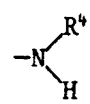

aufweist, oder

$R^1$ und $R^2$ zusammen mit dem Stickstoffatom, an das sie gebunden sind, einen Rest der Formel (III)

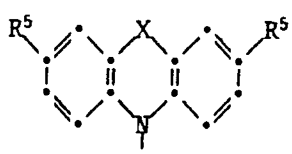

(III)

darstellen,

$R^3$ für einen Rest der Formeln $-(CH_2)_b-COOR^6$ oder $-(CH_2)_2OCOR^7$ steht,

a die Zahl 1, 2 oder 3 und b die Zahl 1 oder 2, und

$R^4$ Phenyl oder eine Gruppe der Formel (II) bedeuten, beide

$R^5$ unabhängig voneinander -H, $C_1$-$C_{24}$-Alkyl, $C_5$-$C_{12}$-Cycloalkyl oder $C_7$-$C_9$-Aralkyl, und

$R^6$ -H, $C_1$-$C_{24}$-Alkyl oder Benzyl bedeuten,

$R^7$ $C_1$-$C_{14}$-Alkyl, Phenyl oder eine Gruppe der Formel

$-(CH_2)_m-Y-(CH_2)_m-COO(CH_2)_2-S-(CH_2)_aN(R^1)(R^2)$ bedeutet,

wobei

Y für -O- oder -S- steht, a die Zahl 1, 2 oder 3 und m die Zahl 1 oder 2 sind,

B eine direkte Bindung, -S-, -S-S- oder einen $C_1$-$C_{12}$-Alkylenrest darstellt,
X in der Formel (III) eine direkte Bindung, -S- oder eine Gruppe der Formeln

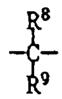 oder 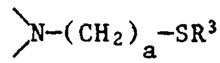

bedeutet,
wobei a und $R^3$ die oben angegebene Bedeutung haben, und
$R^8$ und $R^9$ unabhängig voneinander -H, $C_1$-$C_8$-Alkyl oder Phenyl bedeuten.

Bedeuten $R^1$, $R^8$ und $R^9$ $C_1$-$C_8$-Alkyl, so handelt es sich dabei um geradkettige oder verzweigte Alkylgruppen, beispielsweise um Methyl, Aethyl, n-Propyl, Isopropyl, n-Butyl, tert.-Butyl, tert.-Amyl, n-Hexyl, n-Octyl und 1,1,3,3-Tetrametylbutyl.

Als Beispiele für $C_1$-$C_{14}$-Alkyl als $R^7$ kommen die gleichen Alkylgruppen in Frage, die bereits oben für die Ausdeutung von Alkyl bei $R^1$, $R^8$ und $R^9$ angegeben sind, sowie n-Decyl, n-Dodecyl und n-Tetradecyl.

Bedeuten $R^1$ und $R^5$ $C_7$-$C_9$-Aralkyl, so handelt es sich dabei beispielsweise um Benzyl, $\alpha$-Methylbenzyl oder $\alpha,\alpha$-Dimethylbenzyl.

$C_5$-$C_{12}$-Cycloalkyl als $R^1$ und $R^5$ ist beispielsweise Cyclopentyl, Cyclohexyl, Cyloheptyl, Cylooctyl und Cyclododecyl.

Als Alkaryl mit 7 bis 30 Kohlenstoffatomen stehen R, $R^1$ und $R^2$ z.B. für Phenyl, das durch eine oder zwei geradkettige oder verzweigte Alkylgruppen mit mindestens 1 C-Atom substituiert ist, oder für Naphthyl, das durch eine oder zwei geradkettige oder verzweigte Alkylgruppen mit mindestens 1 C-Atom substituiert ist, wobei die gesamte Zahl der C-Atome der Alkylgruppe(n) höchstens 24 (bei R, $R^1$ und $R^2$ = Phenyl) und 20 (bei R, $R^1$ und $R^2$ = Naphthyl) beträgt. Beispiele für Alkylgruppen sind Methyl, Aethyl, Propyl, Isopropyl, n-Butyl, Isobutyl, tert.-Amyl, n-Hexyl, 1,1,3,3-Tetramethylbutyl, n-Octyl, 2-Aethylhexyl (Isooctyl), n-Decyl, n-Dodecyl, n-Tridecyl, n-Tetradecyl, n-Hexadecyl, n-Octadecyl und n-Eicosyl.

Als Beispiele für $C_1$-$C_{18}$-Alkyl bzw. $C_1$-$C_{24}$-Alkyl als $R^6$ bzw. $R^5$ kommen die gleichen Alkylgruppen in Frage, die bereits oben für die Ausdeutung von Alkylsubstituenten in R, $R^1$ und $R^2$ = Alkaryl angegeben sind. Bevorzugtes Alkyl für $R^6$ ist $C_1$-$C_{16}$-Alkyl, insbesonders $C_4$-$C_{16}$-Alkyl und ganz besonders $C_8$-$C_{14}$-Alkyl.

Bevorzugtes Alkyl für $R^5$ ist Alkyl mit 1-12, insbesonders 4-8 und ganz besonders 8 C-Atomen.

Stellt $R^2$ Phenyl dar, das durch eine $C_1$-$C_{18}$-Alkoxygruppe substituiert ist, so handelt sich bei der Alkoxygruppe beispielsweise um Methoxy, Aethoxy, Propoxy, Isopropoxy, n-Butoxy, Isobutoxy, n-Hexyloxy, n-Octyloxy, n-Decyloxy, n-Dodecyloxy und n-Octadecyloxy.

In der Formel III können die beiden Reste $R^5$ gleich oder verschieden, insbesonders aber gleich, sein.

Bedeutet B $C_1$-$C_{12}$-Alkylen, so handelt es sich dabei um geradkettige oder verzweigte Reste, z.B. Methylen, Aethylen, 1,2-Propylen, 1,3-Propylen, 1,2-, 1,3- oder 1,4-Butylen, 1,5-Pentylen, 2-Methyl-1,3-propylen, 1,6-Hexylen, 1,7-Heptylen, 1,2- oder 1,8-Octylen und 1,10-Decylen.

Erfindungsgemäss bevorzugte Verbindungen weisen die Formel (I) auf, worin
R -H, und
$R^1$ -H, $C_1$-$C_{18}$-Alkyl, $C_7$-$C_{18}$-Alkaryl, Cyclohexyl, Phenyl, Naphthyl oder eine Gruppe der Formel (II)

$-(CH_2)_a-SR^3$ (II) bedeuten,

$R^2$ $C_7$-$C_{18}$-Alkaryl, Phenyl, Naphthyl oder Phenyl ist, das in para-Stellung eine HO-Gruppe oder eine Gruppe der Formel

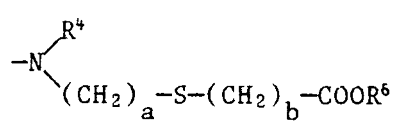

aufweist, oder
$R^1$ und $R^2$ zusammen mit dem Stickstoffatom, an das sie gebunden sind, einen Rest der obigen

Formel (III) darstellen,

R³ für einen Rest der Formel $-(CH_2)_b-COOR^6$ steht,

a die Zahl 1 oder 3 und b die Zahl 1 oder 2 sind,

R⁴ Phenyl oder eine Gruppe der Formel $-(CH_2)_a-S(CH_2)_b-COOR^6$ bedeutet, wobei a und b die oben angegebene Bedeutung haben, und

$R^6$ $C_1-C_{24}$-Alkyl ist, und in der Formel (III) beide

$R^5$ -H, $C_1-C_{12}$-Alkyl oder $C_9$-Phenylalkyl und

X -S- bedeuten.

Naphthyl als $R^1$ und $R^2$ ist $\beta$- und insbesondere $\alpha$-Naphthyl.

$C_9$-Phenylalkyl ist z.B. $\alpha,\alpha$-Dimethylbenzyl oder $\alpha$-Methyl-$\beta$-phenyläthyl.

Besonders bevorzugte Verbindungen der Formel (I) weisen die Formel (I) auf, worin

R -H,

$R^1$ -H, $-CH_3$, $-C_6H_5$, Cyclohexyl, durch $C_4-C_{12}$-Alkyl substituiertes Phenyl oder eine Gruppe der Formel $-CH_2-S-CH_2-COOR^6$, und

$R^2$ $-C_6H_5$, Naphthyl oder durch $C_4-C_{12}$-Alkyl substituiertes Phenyl bedeuten, oder

$R^1$ und $R^2$ zusammen mit dem Stickstoffatom, an das sie gebunden sind, einen Rest der obigen Formel (III) bilden,

a die Zahl 1 ist,

$R^3$ einen Rest der Formel $-CH_2COOR^6$, und

$R^6$ $C_4-C_{18}$-Alkyl bedeuten, und in der Formel (III) beide

$R^5$ -H oder $C_4-C_8$-Alkyl, und

X -S- bedeuten.

$C_4-C_{12}$-Alkyl als Substituent für Phenyl in $R^1$ und $R^2$ ist z.B. n-Butyl, Isobutyl, tert.-Amyl, n-Hexyl, n-Octyl, 2-Aethylhexyl, 1,1,3,3-Tetramethylbutyl, n-Decyl oder n-Dodecyl.

Ganz besonders bevorzugte Verbindungen weisen die Formel (I) auf, worin

R -H,

$R^1$ -H, $CH_3$, $-C_6H_5$, Cyclohexyl, Phenyl enthaltend eine Alkylgruppe mit 8 C-Atomen, oder eine Gruppe der Formel $-CH_2-S-CH_2COOR^6$, und

$R^2$ $-C_6H_5$, Naphthyl oder Phenyl enthaltend eine Alkylgruppe mit 8 C-Atomen bedeuten, oder

$R^1$ und $R^2$ zusammen mit dem Stickstoffatom, an das sie gebunden sind, einen Rest der obigen Formel (III) bilden,

a die Zahl 1 ist,

$R^3$ einen Rest der Formel $-CH_2COOR^6$, und

$R^6$ $C_8-C_{14}$-Alkyl oder beispielsweise ein Gemisch von $C_8H_{17}$-Isomeren bedeuten, und in der Formel (III) beide

$R^5$ -H oder Octyl, und

X -S- bedeuten.

Phenyl enthaltend eine Alkylgruppe mit 8 C-Atomen als $R^1$ und $R^2$ ist z.B. para-(1,1,3,3-Tetramethylbutyl)phenyl.

Octyl als $R^5$ ist z.B. n-Octyl und insbesonders 2-Aethylhexyl.

Zu den ganz besonders bevorzugten Verbindungen gehören solche der Formeln:

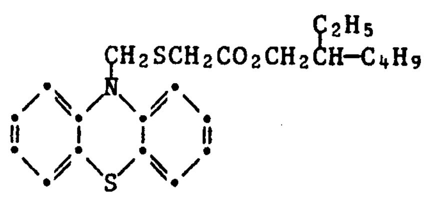

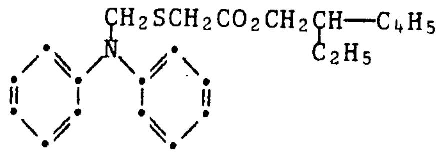

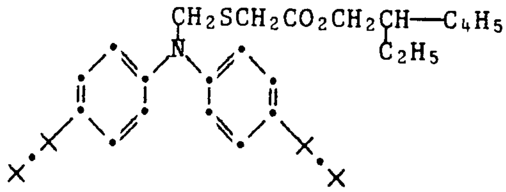

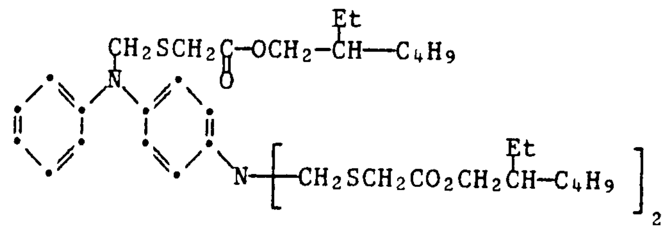

und

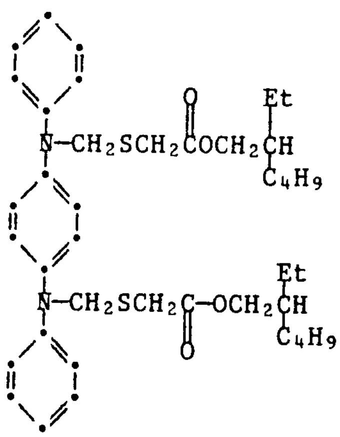

Verbindungen der Formel (I), worin R -H ist, können z.B. hergestellt werden, indem man eine Verbindung der Formel (IV)

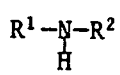

(IV),

worin $R^1$ und $R^2$ die in der Formel (I) angegebene Bedeutung haben, mit Formaldehyd oder einer Formaldehyd abgebenden Verbindung und einer Verbindung der Formel (V)

$R^3SH$ (V) umsetzt,

wobei $R^3$ die in der Formel (I) angegebene Bedeutung hat.

Die Umsetzung kann beispielsweise bei Temperaturen von 15 °C bis 220 °C, insbesonders zwischen 80 °C und 150 °C, in Ab- oder Anwesenheit organischer Lösungsmittel durchgeführt werden.

Als Lösungsmittel eignen sich beispielsweise primäre, sekundäre oder tertiäre Alkohole mit 1 bis 10 C-Atomen, wie Methanol, Aethanol, n-Propanol, Isopropanol, n-Butanol, sek.-Butanol, tert.-Butanol oder n-Pentanol, Ketone, wie Aceton, Methyläthylketon oder Cyclohexanon, Glykole, wie Aethylenglykol oder Diäthylenglykol, ferner Aether, wie Tetrahydrofuran oder Dioxan, oder Glykoläther, wie Aethylenglykol-mono- oder -di-methyläther, Aethylenglykol-mono- oder -diäthyläther, Diäthylenglykol-monomethyläther

oder Diäthylenglykol-monoäthyläther, ferner dipolar-aprotische Lösungsmittel, wie Acetonitril, Benzonitril, Dimethylformamid, N,N-Dimethylacetamid, Nitrobenzol, N-Methylpyrrolidon, aliphatische oder aromatische Kohlenwasserstoffe, wie Hexan, Methylenchlorid, Benzol oder durch Alkyl, Alkoxy oder Halogen substituiertes Benzol, wie Toluol, Xylole, Anisol oder Chlorbenzol, oder aromatische N-Heterocyclen, wie Pyridin, Picolin oder Chinolin, oder Gemische oben aufgeführter Lösungsmittel.

Als Formaldehyd oder Formaldehyd abgebende Verbindung kann man Paraformaldehyd oder wässrige Formaldehydlösungen, wie Formalin, oder Hexamethylentetramin (Urotropin) in Gegenwart einer Base, wie Ammoniak, verwenden.

Sowohl Formaldehyd als die Verbindungen der Formel (V) können in stöchiometrischen Mengen, oder im Ueberschuss, eingesetzt werden. So können sie beispielsweise unabhängig voneinander in einer Menge von 2 bis 10 Mol, bezogen auf 1 Mol des Reaktanden der Formel (IV) eingesetzt werden. Ist mehr als eine NH-Gruppe in der Verbindung der Formel (IV) vorhanden, so sind die Molverhältnisse des Formaldehyds und der Verbindung der Formel (V) entsprechend einzusetzen.

Zur Umsetzung der Reaktanden ist es grundsätzlich möglich, bei tiefere Temperatur alle Komponenten vorzulegen und dann das Gemisch in den Bereich der Reaktionstemperatur zu erwärmen, oder in beliebiger Reihenfolge die einzelnen Komponenten im Bereich der Reaktionstemperatur zueinander zuzugeben. Eine weitere Ausführungsform besteht darin, dass man die Verbindung der Formel (IV) zusammen mit Formaldehyd vorlegt und die Verbindung der Formel (V) im Bereich der Reaktionstemperatur zudosiert. Eine weitere Möglichkeit besteht darin, dass man die Verbindung der Formel (V) und Formaldehyd gleichzeitig zur vorgelegten Verbindung der Formel (IV) zudosiert. Es ist durchaus möglich, das Verfahren nicht nur batchweise, sondern auch kontinuierlich durchzuführen.

Die Verbindungen der Formel (I), worin $R^1$ -H, a die Zahl 1 und R Phenyl, Naphthyl oder $C_7$-$C_{30}$-Alkaryl bedeuten, können z.B. auch hergestellt werden, indem man zuerst ein Aldimin der Formel (VI)

$R^2$-N = CH-R (VI)

durch Umsetzung eines Amins der Formel $R^2NH_2$ mit einem Aldehyd der Formel O = CH-R nach an und für sich bekannten Verfahren herstellt, und dann das so gebildete Aldimin mit einer Verbindung der Formel $HSR^3$ zur Verbindung der Formel (I) umsetzt, wobei R, $R^2$ und $R^3$ die oben angegebene Bedeutung haben.

Die Verbindungen der Formel (I), worin R -H ist und $R^3$ für einen Rest der Formel $-(CH_2)_2OCOR^7$ steht, können z.B. auch hergestellt werden, indem man eine Verbindung der Formel (VII)

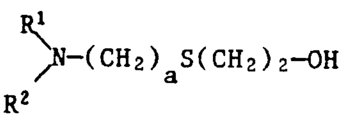 (VII)

mit einem Säurechlorid der Formel $R^7$-COCl, einer Säure der Formel $R^7$-COOH oder einem Säuremethylester der Formel $R^7$-$COOCH_3$, nach an und für sich bekannten Verfahren umsetzt, worin $R^1$, $R^2$, $R^7$ und a die oben angegebene Bedeutung haben.

Die Verbindungen der Formel (VII) können z.B. durch Umsetzung eines Amins der Formel

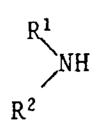

mit Formaldehyd und Mercaptoethanol
nach an und für sich bekannten Verfahren hergestellt werden.

Die Verbindungen der Formel (I), worin a die Zahl 2 oder 3 und R -H bedeuten, können z.B. auch hergestellt werden, indem man ein Thioderivat der Formel (V) an eine Verbindung der Formel (VIII)

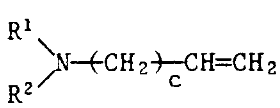 (VIII),

worin c die Zahl 0 oder 1 bedeutet, beispielsweise in Gegenwart von Katalysatoren oder unter Bestrahlung radikalisch addiert, wobei in den Formeln (V) und (VIII) $R^1$, $R^2$ und $R^3$ die oben angegebene Bedeutung haben.

Verbindungen der Formel (I) mit a = 2 oder 3 und R = Phenyl, Naphthyl oder $C_7$-$C_{30}$-Alkaryl können z.B. auch nach der gleichen oben beschriebenen radikalischen Addition hergestellt werden.

Die Verbindungen der Formel (VIII), worin c die Zahl 1 bedeutet, können z.B. durch Kondensation einer Verbindung der Formel

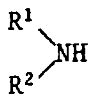

mit einem Allylbromid oder -chlorid in Gegenwart einer Base, wie Kalium- oder Natriumhydroxid oder Natriummethanolat, hergestellt werden. Zur Herstellung der Verbindungen der Formel (I) mit a = 3 und R = Phenyl, Naphthyl oder $C_7$-$C_{30}$-Alkaryl kann z.B. ein durch Phenyl, Naphthyl oder $C_7$-$C_{30}$-Alkaryl substituiertes Allylbromid oder -chlorid eingesetzt werden.

Verbindungen der Formel (VIII), worin c die Zahl 0 ist, können z.B. durch Umsetzung von Vinylacetat mit einer Verbindung der Formel $R^1$-NH-$R^2$ in Gegenwart eines geeigneten Katalysators, z.B. Quecksilberacetat, und Schwefelsäure hergestellt werden. Aus den durch Phenyl, Naphthyl oder $C_7$-$C_{30}$-Alkaryl substituierten Vinylacetatderivaten können die Verbindungen der Formel (I) mit a = 2 und R = Phenyl, Napthyl oder $C_7$-$C_{30}$-Alkaryl nach der gleichen oben aufgeführten Umsetzung gewonnen werden.

Die Verbindungen der Formel (I) eignen sich hervorragend als Zusätze zu Schmiermitteln und Hydraulikflüssigkeiten. Ein weiterer Gegenstand der Erfindung ist daher die Verwendung der Verbindungen der Formel (I) als Additive in Schmiermitteln und Hydraulikflüssigkeiten. Der Zusatz der erfindungsgemässen Verbindungen führt zu einer Verbesserung der allgemeinen Gebrauchseigenschaften, insbesondere der Antioxidans-, Hochdruck- und Antiverschleisseigenschaften. Da die Verbindungen keinen Phosphor enthalten, sind sie besonders für Motorenöle geeignet, da eine Schädigung von Katalysatoren vermieden werden kann. In wässrigen Systemen besteht durch die Abwesenheit von Phosphor eine geringere Gefahr des Befalls mit Mikroorganismen.

Die Verbindungen der Formel (I) werden den Schmiermitteln und Hydraulikflüssigkeiten zweckmässig in einer Menge von 0,01 bis 10 Gew.%, vorzugsweise in einer Menge 0,05 bis 5 Gew.-%, bezogen auf das Schmiermittel oder die Hydraulikflüssigkeit, zugesetzt. In organischen Systemen werden vorteilhaft 0,1 bis 2 Gew.-% und in wässrigen Systemen vorteilhaft 0,05 bis 5 Gew.-% verwendet.

Solche Schmier- und Hydrauliksysteme können von polarer oder umpolarer Natur sein. Die Auswahlkriterien ergeben sich aus den Löslichkeitseigenschaften der entsprechenden Verbindungen.

Die in Frage kommenden Schmiermittel sind dem Fachmann geläufig und z.B. im "Schmiermittel Taschenbuch" (Hüthig Verlag, Heidelberg, 1974) oder von D. Klamann in "Schmierstoffe und verwandte Produkte", Verlag Chemie, Weinheim (1982) beschrieben.

Beispiele hierfür sind Schmiermittel und Hydraulikflüssigkeiten auf Mineralöl-Basis oder synthetische Schmiermittel oder Hydraulikflüssigkeiten, insbesondere die, welche Carbonsäure-Esterderivate darstellen und bei Temperaturen von 200 °C und höher verwendet werden.

Beispiele von synthetischen Schmiermitteln umfassen Schmiermittel auf der Basis eines Diesters einer zweiwertigen Säure mit einem einwertigen Alkohol, wie z.B. Dioctylsebacat oder Dinonyladipat, eines Triesters von Trimethylolpropan mit einer einwertigen Säure oder mit einem Gemisch solcher Säuren, wie z.B. Trimethylolpropantripelargonat, Trimethylolpropan-tricarprylat oder Gemische davon, eines Tetraesters von Pentaerythrit mit einer einwertigen Säure oder mit einem Gemisch solcher Säuren, wie z.B. Pentaerythrit-tetracaprylat, oder eines komplexen Esters von einwertigen und zweiwertigen Säuren mit mehrwertigen Alkoholen, wie z.B. ein komplexer Ester von Trimethylopropan mit Capryl- und Sebacinsäure oder von einem Gemisch davon.

Besonders geeignet sind neben Mineralölen z.B. Poly-$\alpha$-Olefine, Schmiermittel auf Esterbasis, Phosphate, Glykole, Polyglykole und Polyalkylenglykole, sowie deren Mischungen mit Wasser, und Wasser selbst, welches bevorzugt zur Erhöhung der Viskosität zusätzlich noch einen Verdicker enthält.

Die Schmiermittel können zusätzlich andere Additive enthalen, die zugegeben werden, um die Grundeigenschaften von Schmierstoffen noch weiter zu verbessern. Dazu gehören Antioxidantien, Metallpassivatoren, Rostinhibitoren, Viskositätsindex-Verbesserer, Stockpunkterniedriger, Dispergiermitel, Detergentien, Verdicker, Biozide, Antischaummittel, De- und Emulgatoren, sowie andere Hochdruck-Zusätze und Reibungsverminderer.

Als besonders vorteilhaft hat sich die Mitverwendung von Diphenylamin-Additiven der Formel

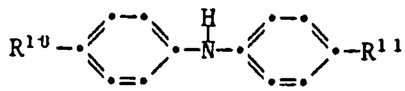

erwiesen,
worin $R^{10}$ und $R^{11}$ unabhängig voneinander -H oder $C_1$-$C_{12}$-Alkyl bedeuten.

$C_1$-$C_{12}$-Alkyl als $R^{10}$ und $R^{11}$ kann geradkettiges oder verzweigtes Alkyl sein. Als Beispiele hierfür kommen die gleichen Beispiele in Frage, welche bereits für die Ausdeutung des Restes $R^1$ als $C_1$-$C_8$-Alkyl angegeben sind, sowie n-Decyl und n-Dodecyl.

Weitere Beispiele für zusätzliche Additive für Schmiermittel und Hydraulikflüssigkeiten sind:

## Beispiele für phenolische Antioxidantien

### 1. Alkylierte Monophenole

2,6-Di-tert-butyl-4-methylphenol, 2,6-Di-tert-butylphenol, 2-tert-Butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclophentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, o-tert-Butylphenol.

### 2. Alkylierte Hydrochinone

2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol.

### 3. Hydroxylierte Thiodiphenylether

2,2′-Thio-bis-(6-tert-butyl-4-methylphenol), 2,2′-Thio-bis(4-octylphenol), 4,4′-Thio-bis-(6-tert-butyl-3-methylphenol), 4,4′-Thio-bis-(6-tert-butyl-2-methylphenol).

### 4. Alkyliden-Bisphenole

2,2′-Methylen-bis-(6-tert-butyl-4-methylphenol), 2,2′-Methylen-bis-(6-tert-butyl-4-ethylphenol), 2,2′-Methylen-bis-[4-methyl-6-(α-methylcyclohexyl)-phenol], 2,2′-Methylen-bis-(4-methyl-6-cyclohexylphenol), 2,2′-Methylen-bis-(6-nonyl-4-methylphenol), 2,2′-Methylen-bis(4,6-di-tert-butylphenol), 2,2′-Ethyliden-bis-(4,6-di-tert-butylphenol), 2,2′-Ethyliden-bis-(6-tert-butyl-4- oder -5-iso-butylphenol), 2,2′-Methylen-bis-[6-(α-methylbenzyl)-4-nonylphenol], 2,2′-Methylen-bis-[6-(α,α-dimethylbenzyl)-4-nonylphenol], 4,4′-Methylen-bis-(2,6-di-tert-butylphenol), 4,4′-Methylenbis-(6-tert-butyl-2-methylphenol), 1,1-Bis-(5-tert-butyl-4-hydroxy-2-methylphenyl)-butan, 2,6-Di-(3-tert-butyl-5-methyl-2-hydroxybenzyl)-4-methylphenol, 1,1,3-Tris-(5-tert-butyl-4-hydroxy-2-methylphenyl)-3-n-dodecylmercaptobutan, Ethylenglycol-bis-[3,3-bis-(3′-tert-butyl-4′-hydroxyphenyl)-butyrat], Bis-(3-tert-butyl-4-hydroxy-5-methylphenyl)-dicyclopentadien, Bis-[2-(3′-tert-butyl-2′-hydroxy-5′-methyl-benzyl)-6-tert-butyl-4-methyl-phenyl]-terephthalat.

### 5. Benzylverbindungen

1,3,5-Tri-(3,5-di-tert-butyl-4-hydroxybenzyl)-2,4,6-trimethylbenzol, Bis-(3,5-di-tert-butyl-4-hydroxybenzyl)-sulfid, 3,5-Di-tert-butyl-4-hydroxybenzyl-mercaptoessigsäure-isooctylester, Bis-(4-tert-butyl-3-hydroxy-2,6-dimethylbenzyl)-dithiol-terephthalat, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat,3,5-Di-tert-butyl-4-hydroxybenzyl-phosphonsäure-dioctadecylester, 3,5-Di-tert-butyl-4-hydroxybenzyl-phosphonsäure-monoethylester, Calcium-salz.

### 6. Acylaminophenole

4-Hydroxy-laurinsäureanilid, 4-Hydroxy-stearinsäureanioid, 2,4-Bis-octylmercapto-6-(3,5-di-tert-butyl-4-hydroxyanilino)-s-triazin, N-(3,5-di-tert-butyl-4-hydroxyphenyl)-carbaminsäureoctylester.

7. Ester der β-(3,5-Di-tert-butyl-4-hydroxyphenyl)-propionsäure

mit ein- oder mehrwertigen Alkoholen, wie z.B. mit Methanol, Diethylenglycol, Octadecanol, Triethylenglycol, 1,6-Hexandiol, Pentaerythrit, Neopentylglycol, Tris-hydroxyethyl-isocyanurat, Thiodiethylenglycol, Bis-hydroxyethyl-oxalsäurediamid.

8. Ester der β-(5-tert-butyl-4-hydroxy-3-methylphenyl)-propionsäure

mit ein- oder mehrwertigen Alkoholen, wie z.B. mit Methanol, Diethylenglycol, Octadecanol, Triethylenglycol, 1,6-Hexandiol, Pentaerythrit, Neopentylglycol, Tris-hydroxyethylisocyanurat, Thiodiethylenglycol, Di-hydroxyethyl-oxalsäurediamid.

9. Amide der β-(3,5-Di-tert-butyl-4-hydroxyphenyl)-propionsäure,

wie z.B. N,N′-Bis-(3,5-di-tert-butyl-4-hydroxyphenylpropionyl)-hexamethylendiamin, N,N′-Bis-(3,5-di-tert-butyl-4-hydroxyphenylpropionyl)-trimethylendiamin, N,N′-Bis-(3,5-di-tert-butyl-4-hydroxyphenylpropionyl)-hydrazin.

Beispiele für aminische Antioxidantien:

N,N′-Di-isopropyl-p-phenylendiamin, N,N′-Di-sec-butyl-p-phenylendiamin, N,N′-Bis(1,4-dimethyl-penthyl)-p-phenylendiamin, N,N′-Bis(1-ethyl-3-methyl-pentyl)-p-phenylendiamin, N,N′-Bis(1-methyl-heptyl)-p-phenylendiamin, N,N′-Dicyclohexyl-p-phenylendiamin, N,N′-Diphenyl-p-phenylendiamin, N,N′-Di-(naphthyl-2)-p-phenylendiamin, N-Isopropyl-N′-phenyl-p-phenylendiamin, N-(1,3-Dimethyl-butyl)-N′-phenyl-p-phenylendiamin, N-(1-Methyl-heptyl)-N′-phenyl-p-phenylendiamin, N-Cyclohexyl-N′-phenyl-p-phenylendiamin, 4-(p-Toluol-sulfonamido)-diphenylamin, N,N′-Dimethyl-N,N′-di-sec-butyl-p-phenylendiamin, Diphenylamin, N-Allyldiphenylamin, 4-Isopropxy-diphenylamin, N-Phenyl-1-naphthylmin, N-Phenyl-2-naphthylamin, octyliertes Diphenylamin, z.B. p,p′-Di-tert-octyldiphenylamin, 4-n-Butylaminophenol, 4-Butyrylamino-phenol, 4-Nonanoylamino-phenol, 4-Dodecanoylamino-phenol, 4-Octadecanoylamino-phenol, Di-(4-methoxy-phenyl)-amin, 2,6-Di-tert-butyl-4-dimethylamino-methyl-phenol, 2,4′-Di-amino-diphenylmethan, 4,4′-Diamino-diphenylmethan, N,N,N′,N′-Tetramethyl-4,4′-diamino-diphenylmethan, 1,2-Di-[(2-methyl-phenyl)-amino]-ethan, 1,2-Di-(phenylamino)-propan, (o-Tolyl)-biguanid, Di-[4-(1′,3′-dimethyl-butyl)-phenyl]amin, tert-octyliertes N-Phenyl-1-naphthylamin, Gemisch aus mono- und dialkylierten tert-Butyl-/tert-Octyldiphenylaminen, 2,3-Dihydro-3,3-dimethyl-4H-1,4-benzothiazin, Phenothiazin, N-Allylphenothiazin.

Beispiele für weitere Antioxidantien:

Aliphatische oder aromatische Phosphite, Ester der Thiodipropionsäure oder der Thiodiessigsäure, oder Salze der Dithiocarbamid- oder Dithiophosphorsäure.

Beispiele für Metall-Desaktivatoren, z.B. für Kupfer, sind:

Triazole, Benztriazole und deren Derivate, Tolutriazole und deren Derivate, 2-Mercaptobenzthiazol, 2-Mercaptobenztriazol, 2,5-Dimercaptobenztriazol, 2,5-Dimercaptobenzthiadiazol, 5,5′-Methylenbisbenztriazol, 4,5,6,7-Tetrahydrobenztriazol, Salicyliden-propylendiamin, Salicylaminoguanidin und dessen Salze.

Beispiele für Rost-Inhibitoren sind:

a) Organische Säuren, ihre Ester, Metallsalze und Anhydride, z.B.: N-Oleoyl-sarcosin, Sorbitan-monooleat, Blei-naphthenat, Alkenylbernsteinsäurenanhydrid, z.B. Dodecenylbernsteinsäure-anhydrid, Alkenylbernsteinsäure-Teilester und -Teilamide, 4-Nonylphenoxyessigsäure.
b) Stickstoffhaltige Verbindungen, z.B.:
I. Primäre, sekundäre oder tertiäre aliphatische oder cycloaliphatische Amine und Amin-Salze von organischen und anorganischen Säuren, z.B. öllösliche Alkylammoniumcarboxylate.
II. Heterocyclische Verbindungen, z.B.:
Substituierte Imidazoline und Oxazoline.
c) Phosphorhaltige Verbindungen, z.B.:
Aminsalze von Phosphorsäurepartialestern oder Phosphonsäurepartialestern, Zinkdialkyldithiophosphate.

d) Schwefelhaltige Verbindungen, z.B.:
Barium-dinonylnaphthalin-sulfonate, Calciumpetroleum-sulfonate.

Beispiele für Viskositätsindex-Verbesserer sind:

Polyacrylate, Polymethacrylate, Vinylpyrrolidon/Methacrylat-Copolymere, Polyvinylpyrrolidone, Polybutene, Olefin-Copolymere, Styrol/Acrylat-Copolymere, Polyether.

Beispiele für Stockpunkterniedriger sind:

Polymethacrylat, alkylierte Naphthalinderivate.

Beispiele für Dispergiermittel/Tenside sind:

Polybutenylbernsteinsäureamide oder -imide, Polybutenylphosphonsäurederivate, basische Magnesium-, Calcium-, und Bariumsulfonate und -phenolate.

Beispiele für Verschleissschutz-Additive sind:

Schwefel und/oder Phosphor und/oder Halogen enthaltende Verbindungen, wie geschwefelte pflanzliche Oele, Zinkdialkyldithiophosphate, Tritolyl-phosphate, chlorierte Paraffine, Alkyl- und Aryldi- und tri-sulfide, Triphenylphosphorothionate, Diethanolaminomethyltolyltriazol, Di(2-ethylhexyl)aminomethyltolyltriazol.

Erfindungsgemäss können die Verbindungen der Formel (I) auch zur Stabilisierung von Elastomeren verwendet werden. Beispiele für Elastomere sind Natur- und synthetische Kautschuke, sowie Gemische davon. Beispiele von Elastomeren sind:
Polydiene, wie beispielsweise Polybutadien, Polyisopren oder Polychloropren; Blockpolymere, wie beispielsweise Styrol/Butadien/Styrol, Styrol/Isopren/Styrol oder Acrylnitril/Butadien-Copolymere;
Copolymere von Mono- und Diolefinen untereinander oder mit anderen Vinylmonomeren, wie z.B. Aethylen-Alkylacrylat-Copolymere, Aethylen-Alkylmethacrylat-Copolymere, Aethylen-Vinylacetat-Copolymere sowie Terpolymere von Aethylen mit Propylen und einem Dien, wie Hexadien, Dicyclopentadien oder Aethylidennorbornen;
Halogenhaltige Polymere, wie z.B. Polychloropren, Chlorkautschuk, chloriertes oder chlorsulfoniertes Polyäthylen, Epichlorhydrin-Homo-und -Copolymere, Chlortrifluoroäthylen-Copolymere, Polymere aus halogenhaltigen Vinylverbindungen, wie z.B. Polyvinylidenchlorid, Polyvinylidenfluorid; sowie deren Copolymere, wie Vinylchlorid-Vinylidenchlorid, Vinylchlorid-Vinylacetat oder Vinylidenchlorid-Vinylacetat;
Polyurethane, die sich von Polyäthern, Polyestern und Polybutadien mit endständigen Hydroxylgruppen einerseite und aliphatischen oder aromatischen Polyisocyanaten andererseits ableiten sowie deren Vorprodukte;
Naturkautschuk;
Mischungen (Polyblends) der vorgenannten Polymeren;
Wässrige Emulsionen natürlicher oder synthetischer Kautschuke, wie z.B. Naturkautschuk-Latex oder Latices von carboxylierten Styrol-Butadien-Copolymeren.

Gegebenenfalls liegen diese Elastomere als Latices vor und können als solche stabilisiert werden.

Bevorzugt sind Zusammensetzungen, welche als Elastomer ein Polydien, wie Polybutadien-Kautschuk, ein halogenhaltiges Polymer, wie Polyvinylidenfluorid, oder ein Polyurethan enthalten.

Die Verbindungen der Formel (I) können beispielsweise in einer Menge von 0,01-10 Gew.%, bevorzugt 0,05-5,0 Gew.%, ganz bevorzugt aber von 0,1-4,0 Gew.%, bezogen auf das Elastomer, eingesetzt werden.

Zusätzlich zu den Verbindungen der Formel (I) können die Elastomere andere Additive enthalten. Beispiele für zusätzliche Additive sind die bereits oben unter den Punkten 1 bis 9 aufgeführten Additive, sowie

10.1. Ester der $\beta$-(3,5-Di-tert.butyl-4-hydroxyphenyl)-propionsäure

mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid.

10.2. Ester der $\beta$-(5-tert.-Butyl-4-hydroxy-3-methylphenyl)-propionsäure

mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)-ethyl-isocyanurat, N,N′-Bis-(hydroxyethyl)-oxalsäurediamid.

10.3. Ester der $\beta$-(3,5-Dicyclohexyl-4-hydroxyphenyl)-propionsäure

mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N′-Bis-(hydroxyethyl)-oxalsäurediamid.

10.4. Amide der $\beta$-(3,5-Di-tert.butyl-4-hydroxyphenyl)-propionsäure,

wie z.B. N,N′-Bis-(3,5-di-tert.butyl-4-hydroxyphenylpropionyl)-hexamethylendiamin, N,N′-Bis-(3,5-di-tert.butyl-4-hydroxyphenylpropionyl)-trimethylendiamin, N,N′-Bis-(3,5-di-tert.butyl-4-hydroxyphenylpropionyl)-hydrazin.

11. UV-Absorber und Lichtschutzmittel

11.1. 2-(2′-Hydroxyphenyl)-benztriazole,

wie z.B. das 5′-Methyl-, 3′,5′-Di-tert.butyl-, 5′-tert.Butyl-, 5′-(1,1,3,3-Tetramethylbutyl)-, 5-Chlor-3′,5′-di-tert.butyl-, 5-Chlor-3′-tert.butyl-5′-methyl-, 3′-sec.Butyl-5′-tert.butyl,4′-Octoxy-, 3′,5′-Di-tert.amyl-, 3′,5′-Bis-($\alpha$,$\alpha$-dimethylbenzyl)-Derivat.

11.2. 2-Hydroxybenzophenone,

wie z.B. das 4-Hydroxy-, 4-Methoxy-, 4-Octoxy-, 4-Decyloxy-, 4-Dodecyloxy, 4-Benzyloxy-, 4,2′,4′-Trihydroxy-, 2′-Hydroxy-4,4′-dimethoxy-Derivat.

11.3. Ester von gegebenenfalls substituierten Benzoesäuren,

wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

11.4. Acrylate,

wie z.B. $\alpha$-Cyan-$\beta$,$\beta$-diphenylacrylsäure-ethylester bzw. -isooctylester, $\alpha$-Carbomethoxy-zimtsäuremethylester, $\alpha$-Cyano-$\beta$-methyl-p-methoxy-zimtsäuremethylester bzw. -butylester, $\alpha$-Carbomethoxy-p-methoxy-zimtsäure-methylester, N-($\beta$-Carbomethoxy-$\beta$-cyanovinyl)-2-methyl-indolin.

11.5. Nickelverbindungen,

wie z.B. Nickelkomplexe des 2,2′-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

11.6. Sterisch gehinderte Amine,

wie z.B. Bis-(2,2,6,6-tetramethylpiperidyl)-sebacat, Bis-(1,2,2,6,6-pentamethylpiperidyl)-sebacat, n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N′-Bis-(2,2,6,6-Tetramethyl-4-piperidyl)-hexamethylendiamin und 4-tert.-Octylamin-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1′-(1,2-Ethandiyl)-bis-(3,3,5,5-tetramethyl-piperazinon).

11.7. Oxalsäurediamide,

wie z.B. 4,4′-Di-octyloxy-oxanilid, 2,2′-Di-octyloxy-5,5′-di-tert.butyl-oxanilid, 2,2′-Di-dodecyloxy-5,5′-di-tert.butyl-oxanilid, 2-Ethoxy-2′-ethyl-oxanilid, N,N′-Bis-(3-di-methylaminopropyl)-oxalamid, 2-Ethoxy-5-tert.butyl-2′-ethyloxanilid und dessen Gemisch mit 2-Ethoxy-2′-ethyl-5,4′-di-tert.butyl-oxanilid, Gemische von o-und p-Methoxy- sowie von o- und p-Ethoxy-di-substituierten Oxaniliden.

12. Metalldesaktivatoren,

wie z.B. N,N′-Diphenyloxalsäurediamid, N-Salicylal-N′-salicyloylhydrazin, N,N′-Bis-(salicyloyl)-hydrazin, N,N′-Bis-(3,5-di-tert.butyl-4-hydroxyphenylpropionyl)-hydrazin, 3-Salicyloylamino-1,2,4-triazol, Bis-(benzyliden)-oxalsäuredihydrazid.

13. Phosphite und Phosphonite,

wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butyphenyl)-pentaerythritdiphosphit, Tristearyl-sorbit-triphosphit, Tetrakis-(2,4-di-tert.butylphenyl)-4,4′-biphenylen-diphosphonit, 3,9-Bis-(2,4-di-tert.butylphenoxy)-2,4,8,10-tetraoxa-3,9-diphosphaspiro[5.5]undecan.

14. Peroxidzerstörende Verbindungen,

wie z.B. Ester der β-Thiodipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

15. Polyamidstabilisatoren,

wie z.B. Kupfersalze in Kombination mit Jodiden und/oder Phosphorverbindungen und Salze des zweiwertigen Mangans.

16. Basische Co-Stabilisatoren,

wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

17. Nukleierungsmittel,

wie z.B. 4-tert.Butylbenzoesäure, Adipinsäure, Diphenylessigsäure.

18. Füllstoffe und Verstärkungsmittel,

wie z.B. Calciumcarbonat, Silikate, Glasfasern, Asbest, Talk, Kaolin, Glimmer, Bariumsulfat, Metalloxide und -hydroxide, Russ, Graphit.

19. Sonstige Zusätze,

wie z.B. Weichmacher, Gleitmittel, Emulgatoren, Pigmente, Optische Aufheller, Flammschutzmittel, Antistatika, Treibmittel.

Die nachfolgenden Beispiele erläutern die Erfindung näher. Teile und Prozent sind Gewichtsteile und Gewichtsprozent, sofern es nicht anders angegebenen ist.

Beispiel 1: (N,N-Diphenylaminomethylthioessigsäure)-2-ethylhexylester

33,9 g (0,2 mol) Diphenylamin, 40,9 g (0,2 mol) Thioglykolsäure-2-ethylhexylester und 6,6 g (0,22 mol) Paraformaldehyd werden in einem Sulfierkolben mit Rückflusskühler und mechanischem Rührer (Flügelrührer ~ 350 U/Min.) gemischt. Die Mischung wird auf 120 °C erwärmt und unter leichtem Stickstoffstrom während 3 Stunden bei dieser Temperatur (Innentemperatur) gehalten. Anschliessend wird das erhaltene Gemisch auf 50 °C abgekühlt und mit 50 ml Toluol versetzt. Im Scheidetrichter wird das Gemisch 2 x mit 3 ml Wasser gewaschen. Die organische Phase wird dann am Rotationsverdampfer eingeengt und während einer Stunde bei 80 °C im Hochvakuum (4 Pa; 0,03 Torr) getrocknet.

Man erhält 75,3 g (≙ 97,6 % Ausbeute) eines leicht braunen Oeles der Formel

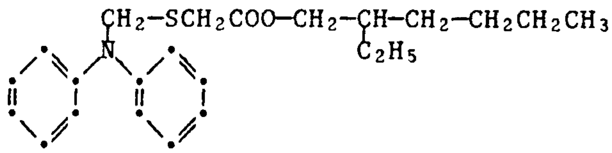

| Analyse ($C_{23}H_{31}NO_2S$; Mol. Gew.: 385,57): | | | | | |
|---|---|---|---|---|---|
| Ber.: | C | 71,65 % | Gef.: | C | 71,5 % |
| | H | 8,10 % | | H | 8,2 % |
| | N | 3,63 % | | N | 3,6 % |
| | S | 8,32 % | | S | 8,4 % |

Beispiel 2: N-(2-Aethylhexyloxycarbonylmethylthiomethyl)-phenothiazin

Setzt man 33,9 g (0,2 mol) Thiodiphenylamin, 40,9 g (0,2 mol) Thioglykolsäure-2-äthylhexylester und 6,6 g (0,22 mol) Paraformaldehyd um, verfährt aber analog dem im obigem Beispiel 1 angegebenen Verfahren, so werden 82,3 g (= 99 % Ausbeute) eines braunen Oeles der Formel

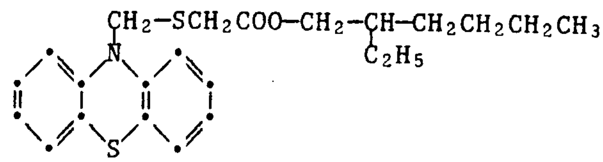

erhalten, welches die folgende Verbrennungsanalyse aufweist:

| Analyse ($C_{23}H_{29}NO_2S_2$; Mol. Gew.: 451,61): | | | | | |
|---|---|---|---|---|---|
| Ber.: | C | 66,47 % | Gef.: | C | 66,2 % |
| | H | 7,03 % | | H | 7,1 % |
| | N | 3,37 % | | N | 3,4 % |
| | S | 15,43 % | | S | 15,6 % |

Beispiel 3: N-Phenyl-N-Naphthylaminomethylthioessigsäure-2-äthylhexylester

Setzt man 43,9 g (0,2 mol) N-Phenyl-N-α-Naphthylamin, 40,9 g (0,2 mol) Thioglykolsäure-2-ethylhexylester und 6,6 g (0,22 mol) Paraformaldehyd um, verfährt man aber analog dem im obigem Beispiel 1 angegebenen Verfahren, so werden 84,3 g (= 96,8 % Ausbeute) eines braunen Oeles der Formel

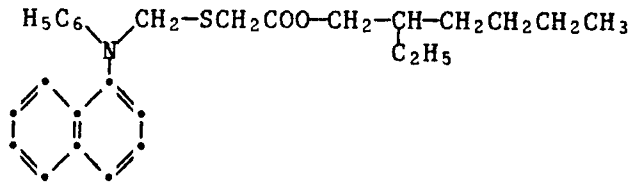

erhalten, welches die folgende Verbrennungsanalyse aufweist:

| Analyse ($C_{27}H_{33}NO_2S$; Mol. Gew.: 435,63): | | | | | |
|---|---|---|---|---|---|
| Ber.: | C | 74,44 % | Gef.: | C | 74,3 % |
| | H | 7,64 % | | H | 7,6 % |
| | N | 3,22 % | | N | 3,2 % |
| | S | 7,36 % | | S | 7,5 % |

Beispiel 4: N-(Methyloxycarboxymethylthiomethyl)-diphenylamin

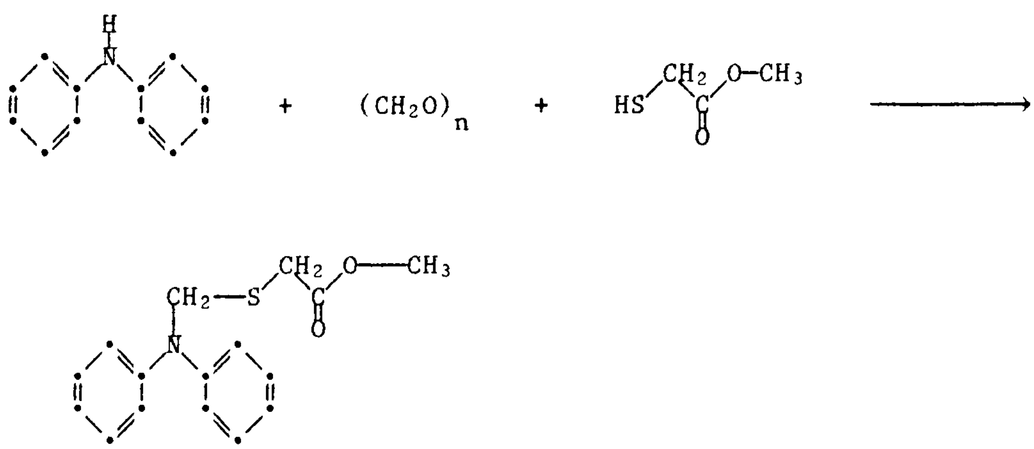

In einem 100 ml Sulfierkolben mit Propellerrührer werden 33,9 g (0,2 mol) Diphenylamin, 6,6 g (0,22 mol) Paraformaldehyd und 21.1 g (0.2 mol) Thioglykolsäuremethylester unter Rühren und unter leichtem Stickstoffatom während 5 Stunden bei 120 °C reagieren gelassen, anschliessend wird das Reaktionsgemisch auf 80 °C abekühlt, in 100 ml Toluol aufgenommen und im Scheidetrichter 3x mit 50 ml $H_2O$ gewaschen. Die organische Phase wird über 10 g $Na_2SO_4$ getrocknet und am Rotationsverdampfer eingeengt und am Hochvakuum bei 80 °C während 1 h getrocknet. Man erhält 56,9 g (99 % d. Theorie) eines braunen Oeles

| | | | | | | | | | |
|---|---|---|---|---|---|---|---|---|---|
| Analyse | gefunden | C | 66.7 %, | H | 6,0 %, | N | 4,9 %, | S | 11,2 % |
| | berechnet | C | 66.87 %, | H | 5,96 %, | N | 4,87 %, | S | 11,16% |

Beispiel 5: N,N-Bis(2-ethylhexyloxycarboxymethylthiomethyl)-naphthylamin

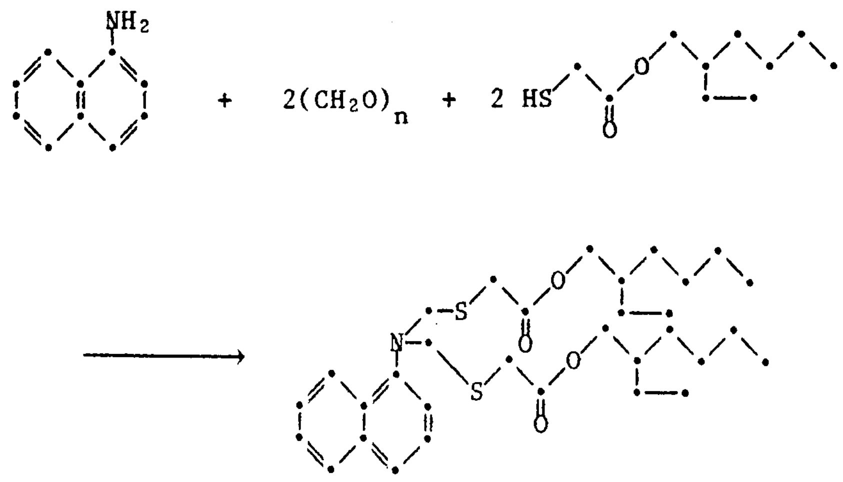

In einem 350 ml Sulfierkolben mit Propellerrührer werden 14,3 g (0,1 mol) 1-Naphthylamin, 6,6 g (0,22 mol) Paraformaldehyde und 40,8 g (0,2 mol) Thioglykolsäure-2-äthylhexylester vorgelegt. Die Reaktionsmischung wird unter Rühren und unter leichtem Stickstoffstrom während 3 Stunden bei 120 °C reagieren gelassen, auf 80 °C abgekühlt, in 200 ml Toluol aufgenommen und im Scheidetrichter 3 x mit 100 ml $H_2O$ gewaschen. Die organische Phase wird über 10 g $Na_2SO_4$ getrocknet, filtriert, am Rotationsverdampfer eingeengt und am Hochvakuum bei 80 °C während 2 h getrocknet.
Man erhält 54,8 g (95 % d. Theo.) eines braunen Oeles.

| | | | | | | | | | |
|---|---|---|---|---|---|---|---|---|---|
| Analyse | berechnet | C | 66,75 %, | H | 8,58 %, | N | 2,43 %, | S | 11,14 % |
| | gefunden | C | 66,9 %, | H | 8,5 %, | N | 2,3 %, | S | 11,2 % |

Beispiel 6: N,N′-Bis(2-ethylhexyloxycarboxymethylthiomethyl)-N,N′-diphenylparaphenylendiamin

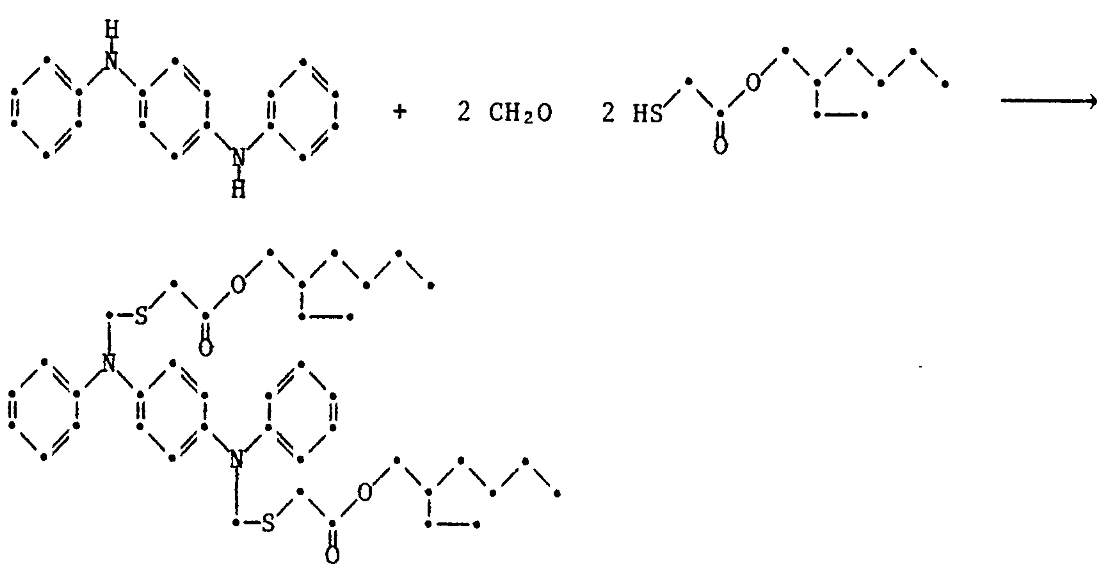

In einem 750 ml Sulfierkolben mit Propellerrührer werden 52,1 g (0,2 mol) N,N′-Diphenyl-1,4-Phenylendiamin und 81,7 g (0,4 mol) Thioglykolsäure-2-ethylhexylester in 200 ml Ethanol vorgelegt und auf Rückflusstemperatur erhitzt. 36,7 g (0,44 mol) Formaldehyd 36 % in Wasser werden innert 2 Stunden

zugetropft und anschliessend wird das Reaktionsgemisch während 16 h am Rückfluss gekocht. Am Rotationsverdampfer wird der Ansatz eingeengt und anschliessend am Hochvakuum bei 80 °C während 12 h getrocknet. Man erhält 127,3 g (91,8 % d. Theorie) eines dunkelbraunen Oeles.

| | | | | | | | | | |
|---|---|---|---|---|---|---|---|---|---|
| Analyse | berechnet<br>gefunden | C<br>C | 69,33 %,<br>70,2 %, | H<br>H | 8,15 %,<br>8,13 %, | N<br>N | 4,04 %,<br>3,96 %, | S<br>S | 9,25 %<br>9,29 % |

Beispiel 7: N,N-Bis(2-ethylhexyloxycarboxymethylthiomethyl)-4-methylanilin

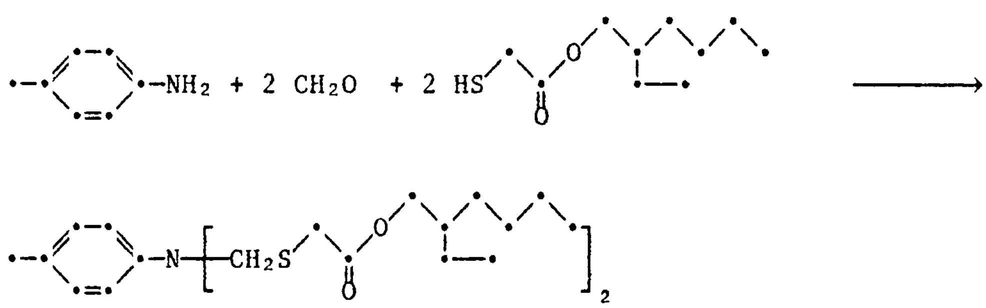

21,4 g (0,2 mol) p-Toluidin und 81,73 g (0,4 mol) Thioglykolsäure-2-äthylhexylester werden in 200 ml Alkohol in einem 750 ml Sulfierkolben unter leichtem Stickstoffstrom vorgelegt und am Rückfluss gekocht. Unter diesen Bedingungen wird innerhalb 1 h 36,7 g (0,44 mol) Formaldehyd 36 % zugetropft. Anschliessend wird während 12 h am Rückfluss kocht.

Die Lösung wird am Rotationsverdampfer eingeengt und der Rückstand am Hochvakuum bei 80 °C während 1 h getrocknet.

Man erhält 104,5 g (96.8 % d. Theorie) eines leicht braunen Oeles.

| | | | | | | | | | |
|---|---|---|---|---|---|---|---|---|---|
| Analyse | gefunden:<br>berechnet: | C<br>C | 64,3 %;<br>64,52 %; | H<br>H | 9,1 %;<br>9,15 %; | N<br>N | 2,7 %;<br>2,59 %; | S<br>S | 12,0 %<br>11,88 % |

Beispiel 8: Test auf Stabilisierung gegen Oxidation
(TFOUT-Test: Thin-Film Oxygen Uptake Test)

Dieser Test ist eine modifizierte Version des "Rotary Bomb Oxidationstests für Mineralöle" (ASTM D 2272). Er wird genau beschrieben in "C.S. Ku und S.M. Hsu, A Thin-Film Oxygen Uptake Test for the Evaluation of Automotive Crankcase Lubricants, Lubrication Engineering, Vol. 40 (2), 75-83 (1984)". Als Testöl dient ein handelsübliches Motorenöl mit eingestelltem Zinkdithiophosphat-Gehalt (ZDTP) auf Mineralölbasis, welches die Hälfte der üblichen Menge an Zinkdithiophosphat (0,75%: Zinkgehalt 0,06 %, bezogen auf das Motorenöl) enthält; diese Aenderung wurde vorgenommen, damit ein potentieller Effekt des zu prüfenden Stabilisators gezeigt werden kann.

Die in Beispielen 1 - 4 hergestellten Additive werden im beschriebenen Motorenöl in Gegenwart von 2 % einer flüssigen oxidierten, nitrierten Fraktion eines Motorenbenzins als Katalysator (4 % Einsatzkonzentration), eines flüssigen Metallnaphthenates, als weiterem Katalysator (4 % Einsatzkonzentration; Wasser und die beiden flüssigen Katalysatorsubstanzen werden unter der No. Standard Reference Material 1817 vom National Bureau of Standards (NBS) mit Analysen-Zertifikat geliefert) getestet. Der Versuch ist bei einem deutlichen Knick des Druck/Zeit-Diagramms beendet. Die in der untenstehenden Tabelle angegebenen Resultate bedeuten die Zeit (in Minuten) bis zum Knick des Druck/Zeit-Diagramms.

Lange Zeiten entsprechen guter Stabilisatorwirksamkeit der Additive. Konzentration des Additives: 0,5 Gew.-%, bezogen auf as Oel.

| Formulierung | | Induktionsperiode (Zeit bis zum deutlichen Druckabfall in Minuten) |
|---|---|---|
| Oel | | 76 |
| Oel | + Additiv nach Beispiel 1 | 140 |
| Oel | + Additiv nach Beispiel 2 | 116 |
| Oel | + Additiv nach Beispiel 3 | 141 |
| Oel | + Additiv nach Beispiel 4 | 160 |

Beispiel 9: Test auf Verschleissschutz

Zur Prüfung auf die Eignung auf Verschleissschutz wird die ASTM-Standard-MethodeD 2783-81 unter Verwendung des Shell-Vierkugelapparates herangezogen. Als Basisöl wird Catenex® P 941 der Fa. Shell verwendet. Ermittelt werden a) die Schweisslast WL (Weld Load) als die last (in kg) bei der die 4 Kugeln innerhalb von 10 Sekunden zusammenschweissen, und b) der mittlere Verschleiss-Durchmesser WSD (Wear Scar Diameter) bei einer Last von 40 kg während 1 Stunde (in mm).

Bei allen Testproben wird 1 Gew.% des Additives eingesetzt.

Die Ergebnisse sind in der Tabelle zusammengefasst.

| Additiv aus Beispiel Nr. | WL (kg) | WSD (mm) |
|---|---|---|
| ohne Additiv (Vergleich) | 160 | 0,90 |
| 1 | 180 | 0,60 |
| 4 | 200 | 0,52 |
| 5 | 200 | 0,55 |

Beispiel 10: Test auf Kupferpassivierung

Ein blank poliertes Kupferblech von 60 x 10 x 1 mm wird in Turbineöl getaucht, das 50 ppm gelösten Schwefel sowie 0,05 % 3-[Bis(2'-ethylhexyl)aminomethyl]-benzothiazolin-2-thion enthält. Eine Vergleichsprobe enthält kein Thiazolinderivat. Die Proben werden 2 Stunden auf 100 °C erwärmt. Anschliessend wird das Kupferblech mit Petrolether gewaschen, getrocknet und die Farbe des Bleches nach ASTM D 130 beurteilt durch Vergleich mit einer Standard-Farbtabelle. Die Beurteilung geschieht in 4 Stufen:

1 - kein Beschlag,
2 - mässiger Beschlag,
3 - starker Beschlag,
4 - Korrosion.

Ergebnis: Farbe der Probe mit dem Additiv gemäss Beispiel 1):
1 (kein Beschlag).

Beispiel 11: Stabilisierung von Polybutadien-Kautschuk

100 Teile Polybutadien, welches mit 0,36 % 2,6-Di-tert.-butyl-p-cresol vorstabilisiert ist, wird zusätzlich mit 0.25 % des Additives auf einem Zweiwalzenstuhl bei 50 °C während 6 Minuten plastifiziert.

Aus den Walzfellen werden bei 60 °C Platten von 10 mm Dicke gepresst. Die Platte ohne die erfindungsgemässe Verbindung wird auf dieselbe Weise hergestellt.

Die Prüfung auf Wirksamkeit des zugesetzten Additivs wird durch Hitzealterung in einem Umluftofen bei 80 °C vorgenommen. Als Kriterium dient der während der Ofenalterung auftretende Gelgehalt. Der Gelgehalt nimmt nach einer Induktionsperiode rasch zu. Als willkürliche Definition der Induktionsperiode dient die Zeit, nach welcher ein Gelgehalt von 5 % erreicht wird.

Der Gelgehalt wird folgendermassen bestimmt:
1 Gramm des Polybutadienes wird während 12 h bei Raumtemperatur in 100 ml Toluol gelöst. Die Lösung wird durch Glaswolle filtriert und die filtrierte Lösung zur Trockene eingedampft. Der Gelgehalt ergibt sich aus:

$$\text{Gel} = \frac{E - A}{E} \quad x \quad 100\ (\%)$$

E = Einwaage (1 Gramm)
A = Gewicht des Eindampfrückstandes
Die Ergebnisse sind in der Tabelle zusammengefasst:

| Additiv aus Beispiel (0.25 %) | Behandlung im Ofen bei 80°C in Anzahl Wochen, bis Gelgehalt 5 % |
|---|---|
| Kontrolle (ohne Additiv) | 4 |
| 1 | 7 |
| 6 | 8 |
| 12 * | 13 |

* Ein Produkt der Formel

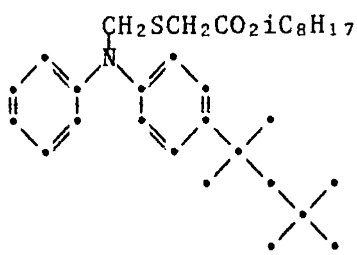

N-(2-Ethylhexyloxycarboxymethylthiomethyl)-N-phenyl-p-isooctylphenylamin hergestellt nach einem Verfahren analog Beispiel 1.

**Patentansprüche**

**Patentansprüche für folgende Vertragsstaaten : AT, BE, DE, FR, GB, IT, NL, SE**

**1.** Verbindungen der Formel (I)

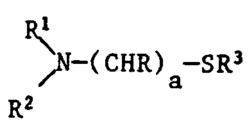

(I),

worin

R unabhängig voneinander -H, Phenyl, Naphthyl oder $C_7$-$C_{30}$-Alkaryl, und

$R^1$ -H, $C_1$-$C_8$-Alkyl, $C_7$-$C_9$-Aralkyl, $C_5$-$C_{12}$-Cycloalkyl, Phenyl, Naphthyl, $C_7$-$C_{30}$-Alkaryl oder eine Gruppe der Formel (II)

$-(CH_2)_a-SR^3$ (II) bedeuten,

$R^2$ $C_7$-$C_{30}$-Alkaryl, Phenyl, Naphthyl oder Phenyl bedeutet, das in para-Stellung eine HO- oder $C_1$-$C_{18}$-Alkoxygruppe oder eine Gruppe der Formeln

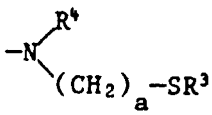
oder
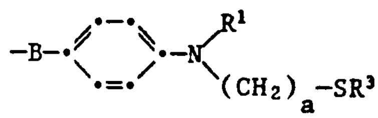
oder
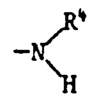

aufweist, oder

$R^1$ und $R^2$ zusammen mit dem Stickstoffatom, an das sie gebunden sind, einen Rest der Formel (III)

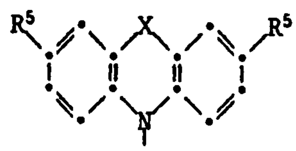
(III)

darstellen,

$R^3$ für einen Rest der Formeln $-(CH_2)_b-COOR^6$ oder $-(CH_2)_2OCOR^7$ steht,

a die Zahl 1, 2 oder 3 und b die Zahl 1 oder 2, und

$R^4$ Phenyl oder eine Gruppe der Formel (II) bedeuten, beide

$R^5$ unabhängig voneinander -H, $C_1$-$C_{24}$-Alkyl, $C_5$-$C_{12}$-Cycloalkyl oder $C_7$-$C_9$-Aralkyl, und

$R^6$ -H, $C_1$-$C_{24}$-Alkyl oder Benzyl bedeuten,

$R^7$ $C_1$-$C_{14}$-Alkyl, Phenyl oder eine Gruppe der Formel

$-(CH_2)_m-Y-(CH_2)_m-COO(CH_2)_2-S-(CH_2)_aN(R^1)(R^2)$ bedeutet,

wobei

Y für -O- oder -S- steht, a die Zahl 1, 2 oder 3 und m die Zahl 1 oder 2 sind,

B eine direkte Bindung, -S-, -S-S- oder einen $C_1$-$C_{12}$-Alkylenrest darstellt,

X in der Formel (III) eine direkte Bindung, -S- oder eine Gruppe der Formeln

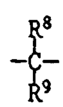
oder
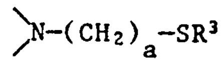

bedeutet,

wobei a und $R^3$ die oben angegebene Bedeutung haben, und

$R^8$ und $R^9$ unabhängig voneinander -H, $C_1$-$C_8$-Alkyl oder Phenyl bedeuten.

**2.** Verbindungen der Formel (I) gemäss Anspruch 1, wobei

R -H, und

$R^1$ -H, $C_1$-$C_{18}$-Alkyl, $C_7$-$C_{18}$-Alkaryl, Cyclohexyl, Phenyl, Naphthyl oder eine Gruppe der Formel (II)

$-(CH_2)_a-SR^3$ (II) bedeuten,

$R^2$ — $C_7$-$C_{18}$-Alkaryl, Phenyl, Naphthyl oder Phenyl ist, das in para-Stellung eine HO-Gruppe oder eine Gruppe der Formel

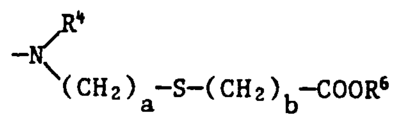

aufweist, oder
$R^1$ und $R^2$ — zusammen mit dem Stickstoffatom, an das sie gebunden sind, einen Rest der Formel (III) gemäss Anspruch 1 darstellen,
$R^3$ — für einen Rest der Formel $-(CH_2)_b-COOR^6$ steht,
a — die Zahl 1 oder 3 und b die Zahl 1 oder 2 sind,
$R^4$ — Phenyl oder eine Gruppe der Formel $-(CH_2)_a-S(CH_2)_b-COOR^6$ bedeutet, wobei a und b die oben im Anspruch 2 angegebene Bedeutung haben, und
$R^6$ — $C_1$-$C_{24}$-Alkyl ist, und in der Formel (III) beide
$R^5$ — -H, $C_1$-$C_{12}$-Alkyl oder $C_9$-Phenylalkyl, und
X — -S- bedeuten.

**3.** Verbindungen der Formel (I) gemäss Anspruch 1,
wobei
R — -H,
$R^1$ — -H, $-CH_3$, $-C_6H_5$, Cyclohexyl, durch $C_4$-$C_{12}$-Alkyl substituiertes Phenyl oder eine Gruppe der Formel $-CH_2-S-CH_2-COOR^6$, und
$R^2$ — $-C_6H_5$, Naphthyl oder durch $C_4$-$C_{12}$-Alkyl substituiertes Phenyl bedeuten, oder
$R^1$ und $R^2$ — zusammen mit dem Stickstoffatom, an das sie gebunden sind, einen Rest der Formel (III) gemäss Anspruch 1 bilden,
a — die Zahl 1 ist,
$R^3$ — einen Rest der Formel $-CH_3COOR^6$, und
$R^6$ — $C_4$-$C_{18}$-Alkyl bedeuten, und in der Formel (III) beide
$R^5$ — -H oder $C_4$-$C_8$-Alkyl, und
X — -S- bedeuten.

**4.** Verbindungen der Formel (I) gemäss Anspruch 1, wobei
R — -H,
$R^1$ — -H, $-CH_3$, $-C_6H_5$, Cyclohexyl, Phenyl enthaltend eine Alkylgruppe mit 8 C-Atomen, oder eine Gruppe der Formel $-CH_2-S-CH_2COOR^6$, und
$R^2$ — $-C_6H_5$, Naphthyl oder Phenyl enthaltend eine Alkylgruppe mit 8 C-Atomen bedeuten, oder
$R^1$ und $R^2$ — zusammen mit dem Stickstoffatom, an das sie gebunden sind, einen Rest der Formel (III) gemäss Anspruch 1 bilden,
a — die Zahl 1 ist,
$R^3$ — einen Rest der Formel $-CH_2COOR^6$, und
$R^6$ — $C_8$-$C_{14}$-Alkyl oder ein Gemisch von $C_8H_{17}$-Isomeren bedeuten, und in der Formel (III) beide
$R^5$ — -H oder Octyl, und
X — -S- bedeuten.

**5.** Zusammensetzung enthaltend
a) mindestens ein Schmiermittel, ein Hydraulikflüssigkeit oder ein Elastomer und
b) 0,01-10 Gew.%, bezogen auf das Schmiermittel, die Hydraulikflüssigkeit oder das Elastomer, mindestens einer Verbindung der Formel (I) gemäss Anspruch 1.

**6.** Zusammensetzung gemäss Anspruch 5, worin die Komponente a) ein Schmiermittel ist.

**7.** Zusammensetzung gemäss Anspruch 5, worin die Komponente a) ein Elastomer ist.

**8.** Verwendung der Verbindungen der Formel (I) gemäss Anspruch 1 als Additive in Schmiermitteln, Hydraulikflüssigkeiten und Elastomeren.

**Patentansprüche für folgenden Vertragsstaat : ES**

**1.** Zusammensetzung enthaltend
a) mindestens ein Schmiermittel, ein Hydraulikflüssigkeit oder ein Elastomer und
b) 0,01-10 Gew.%, bezogen auf das Schmiermittel, die Hydraulikflüssigkeit oder das Elastomer, mindestens einer Verbindung der Formel (I)

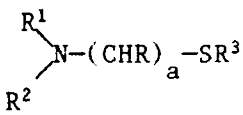

(I),

worin

R unabhängig voneinander -H, Phenyl, Naphthyl oder $C_7$-$C_{30}$-Alkaryl, und

$R^1$ -H, $C_1$-$C_8$-Alkyl, $C_7$-$C_9$-Aralkyl, $C_5$-$C_{12}$-Cycloalkyl, Phenyl, Naphthyl, $C_7$-$C_{30}$-Alkaryl oder eine Gruppe der Formel (II)

$-(CH_2)_a-SR^3$ (II) bedeuten,

$R^2$ $C_7$-$C_{30}$-Alkaryl, Phenyl, Naphthyl oder Phenyl bedeutet, das in para-Stellung eine HO- oder $C_1$-$C_{18}$-Alkoxygruppe oder eine Gruppe der Formeln

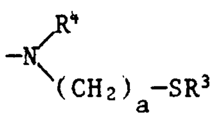 oder 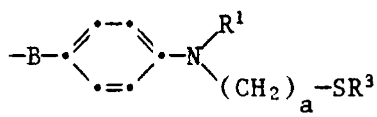 oder 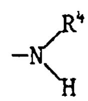

aufweist, oder

$R^1$ und $R^2$ zusammen mit dem Stickstoffatom, an das sie gebunden sind, einen Rest der Formel (III)

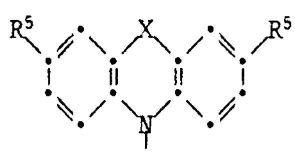

(III)

darstellen,

$R^3$ für einen Rest der Formeln $-(CH_2)_b-COOR^6$ oder $-(CH_2)_2OCOR^7$ steht,

a die Zahl 1, 2 oder 3 und b die Zahl 1 oder 2, und

$R^4$ Phenyl oder eine Gruppe der Formel (II) bedeuten, beide

$R^5$ unabhängig voneinander -H, $C_1$-$C_{24}$-Alkyl, $C_5$-$C_{12}$-Cycloalkyl oder $C_7$-$C_9$-Aralkyl, und

$R^6$ -H, $C_1$-$C_{24}$-Alkyl oder Benzyl bedeuten,

$R^7$ $C_1$-$C_{14}$-Alkyl, Phenyl oder eine Gruppe der Formel

$-(CH_2)_m-Y-(CH_2)_m-COO(CH_2)_2-S-(CH_2)_aN(R^1)(R^2)$ bedeutet,

wobei

Y für -O- oder -S- steht, a die Zahl 1, 2 oder 3 und m die Zahl 1 oder 2 sind,

B eine direkte Bindung, -S-, -S-S- oder einen $C_1$-$C_{12}$-Alkylenrest darstellt,

X in der Formel (III) eine direkte Bindung, -S- oder eine Gruppe der Formeln

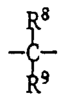 oder 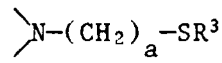

bedeutet,

wobei a und $R^3$ die oben angegebene Bedeutung haben, und

$R^8$ und $R^9$ unabhängig voneinander -H, $C_1$-$C_8$-Alkyl oder Phenyl bedeuten.

**2.** Zusammensetzungen gemäss Anspruch 1, wobei in der Verbindung der Formel (I)

R -H, und

$R^1$ -H, $C_1$-$C_{18}$-Alkyl, $C_7$-$C_{18}$-Alkaryl, Cyclohexyl, Phenyl, Naphthyl oder eine Gruppe der Formel (II)

$-(CH_2)_a-SR^3$ (II) bedeuten,

$R^2$ $C_7$-$C_{18}$-Alkaryl, Phenyl, Naphthyl oder Phenyl ist, das in para-Stellung eine HO-Gruppe oder eine Gruppe der Formel

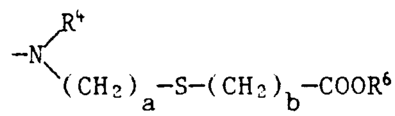

aufweist, oder

$R^1$ und $R^2$ zusammen mit dem Stickstoffatom, an das sie gebunden sind, einen Rest der Formel (III) gemäss Anspruch 1 darstellen,

$R^3$ für einen Rest der Formel $-(CH_2)_b-COOR^6$ steht,

a die Zahl 1 oder 3 und b die Zahl 1 oder 2 sind,

$R^4$ Phenyl oder eine Gruppe der Formel $-(CH_2)_a-S(CH_2)_b-COOR^6$ bedeutet, wobei a und b die oben im Anspruch 2 angegebene Bedeutung haben, und

$R^6$ $C_1$-$C_{24}$-Alkyl ist, und in der Formel (III) beide

$R^5$ -H, $C_1$-$C_{12}$-Alkyl oder $C_9$-Phenylalkyl, und

X -S- bedeuten.

**3.** Zusammensetzung gemäss Anspruch 1, wobei in der Verbindung der Formel (I)

R -H,

$R^1$ -H, $-CH_3$, $-C_6H_5$, Cyclohexyl, durch $C_4$-$C_{12}$-Alkyl substituiertes Phenyl oder eine Gruppe der Formel $-CH_2-S-CH_2-COOR^6$, und

$R^2$ $-C_6H_5$, Naphthyl oder durch $C_4$-$C_{12}$-Alkyl substituiertes Phenyl bedeuten, oder

$R^1$ und $R^2$ zusammen mit dem Stickstoffatom, an das sie gebunden sind, einen Rest der Formel (III) gemäss Anspruch 1 bilden,

a die Zahl 1 ist,

$R^3$ einen Rest der Formel $-CH_2COOR^6$, und

$R^6$ $C_4$-$C_{18}$ bedeuten, und in der Formel (III) beide

$R^5$ -H oder $C_4$-$C_8$-Alkyl, und

X -S- bedeuten.

**4.** Zusammensetzung gemäss Anspruch 1, wobei in der Verbindung der Formel (I)

R -H,

$R^1$ -H, $-CH_3$, $-C_6H_5$, Cyclohexyl, Phenyl enthaltend eine Alkylgruppe mit 8 C-Atomen, oder eine Gruppe der Formel $-CH_5-S-CH_2COOR^6$, und

$R^2$ $-C_6H_5$, Naphthyl oder Phenyl enthaltend eine Alkylgruppe mit 8 C-Atomen bedeuten,

oder

$R^1$ und $R^2$ zusammen mit dem Stickstoffatom, an das sie gebunden sind, einen Rest der Formel (III) gemäss Anspruch 1 bilden,

a die Zahl 1 ist,

$R^3$ einen Rest der Formel $-CH_2COOR^6$, und

$R^6$ $C_8$-$C_{14}$-Alkyl oder ein Gemisch von $C_8H_{17}$-Isomeren bedeuten, und in der Formel (III) beide

$R^5$ -H oder Octyl, und

X -S- bedeuten.

**5.** Zusammensetzung gemäss Anspruch 1, worin die Komponente a) ein Schmiermittel ist.

**6.** Zusammensetzung gemäss Anspruch 1, worin die Komponente a) ein Elastomer ist.

**7.** Verwendung der Verbindungen der Formel (I) gemäss Anspruch 1 als Additive in Schmiermitteln, Hydraulikflüssigkeiten und Elastomeren.

**8.** Verfahren zur Herstellung von in einem der Ansprüche 1 bis 4 definierten Verbindungen der Formel (I), dadurch gekennzeichnet, dass Verbindungen der Formel (I), worin R -H ist, hergestellt werden, indem man eine Verbindung der Formel (IV)

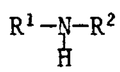 (IV),

worin $R^1$ und $R^2$ die in der Formel (I) in Anspruch 1 angegebene Bedeutung haben, mit Formaldehyd oder einer Formaldehyd abgebenden Verbindung und einer Verbindung der Formel (V)

$R^3SH$ (V) umsetzt,

wobei $R^3$ die in der Formel (I) angegebene Bedeutung hat, oder
die Verbindungen der Formel (I), worin $R^1$ -H, a die Zahl 1 und R Phenyl, Naphthyl oder $C_7$-$C_{30}$-Alkaryl bedeuten, werden hergestellt, indem man zuerst ein Aldimin der Formel (VI)

$R^2$-N = CH-R (VI)

durch Umsetzung eines Amins der Formel $R^2NH_2$ mit einem Aldehyd der Formel O = CH-R nach an und für sich bekannten Verfahren herstellt, und dann das so gebildete Aldimin mit einer Verbindung der Formel $HSR^3$ zur Verbindung der Formel (I) umsetzt, wobei $R^2$ und $R^3$ die in Anspruch 1 angegebene Bedeutung haben, oder
die Verbindungen der Formel (I), worin R -H ist und $R^3$ für einen Rest der Formel $-(CH_2)_2OCOR^7$ steht, werden hergestellt, indem man eine Verbindung der Formel (VII)

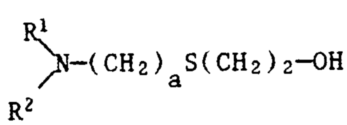 (VII)

mit einem Säurechlorid der Formel $R^7$-COCl, einer Säure der Formel $R^7$-COOH oder einem Säuremethylester der Formel $R^7$-$COOCH_3$, nach an und für sich bekannten Verfahren umsetzt, worin $R^1$, $R^2$, $R^7$ und a die in Anspruch 1 angegebene Bedeutung haben, oder
die Verbindungen der Formel (I), worin a die Zahl 2 oder 3 und R -H bedeuten, werden hergestellt, indem man ein Thioderivat der Formel (V) an eine Verbindung der Formel (VIII)

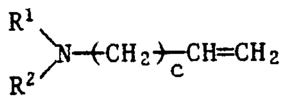

(VIII),

worin c die Zahl 0 oder 1 bedeutet, beispielsweise in Gegenwart von Katalysatoren oder unter Bestrahlung radikalisch addiert, wobei in den Formeln (V) und (VIII) $R^1$, $R^2$ und $R^3$ die in Anspruch 1 angegebene Bedeutung haben, oder
die Verbindungen der Formel (I) mit a = 2 oder 3 und R = Phenyl, Naphthyl oder $C_7$-$C_{30}$-Alkaryl werdem nach der gleichen oben beschriebenen radikalischen Addition hergestellt.

**Claims**

**Claims for the following Contracting States : AT, BE, DE, FR, GB, IT, NL, SE**

**1.** A compound of the formula (I)

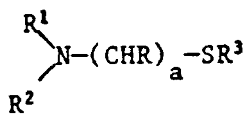

(I)

in which the Rs independently of one another are H, phenyl, naphthyl or $C_7$-$C_{30}$alkaryl and $R^1$ is H, $C_1$-$C_8$alkyl, $C_7$-$C_9$aralkyl, $C_5C_{12}$cyclo-alkyl, phenyl, naphthyl, $C_7$-$C_{30}$alkaryl or a group of the formula (II)

$-(CH_2)_a-SR^3$ (II)

$R^2$ is $C_7$-$C_{30}$alkaryl, phenyl, naphthyl or phenyl containing an HO or $C_1$-$C_{18}$alkoxy group or a group of the formulae

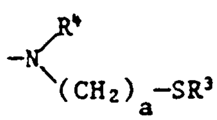 or 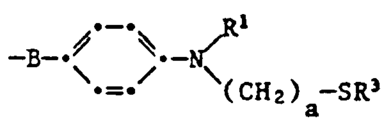 or 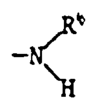

in the para-position, or $R^1$ and $R^2$, together with the nitrogen atom to which they are attached, are a radical of the formula (III)

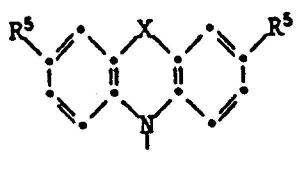

(III)

$R^3$ is a radical of the formulae $-(CH_2)_b-COOR^6$ or $-(CH_2)_2OCOR^7$, a is the number 1, 2 or 3 and b is the number 1 or 2, and $R^4$ is phenyl or a group of the formula (II), the two $R^5$s independently of one another are H, $C_1$-$C_{24}$alkyl, $C_5$-$C_{12}$cycloalkyl or $C_7$-$C_9$aralkyl, and $R^6$ is H, $C_1$-$C_{24}$alkyl or benzyl, $R^7$ is $C_1$-$C_{14}$alkyl, phenyl or a group of the formula

$-(CH_2)_m-Y-(CH_2)_m-COO(CH_2)_2-S-(CH_2)_aN(R^1)(R^2)$

in which Y is -O- or -S-, a is the number 1, 2 or 3 and m is the number 1 or 2, B is a direct bond, -S-, -S-S- or a $C_1$-$C_{12}$alkylene radical, X in the formula (III) is a direct bond, -S- or a group of the formulae

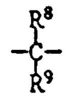

or

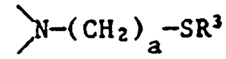

in which a and $R^3$ are as defined above, and $R^8$ and $R^9$ independently of one another are H, $C_1$-$C_8$alkyl or phenyl.

**2.** A compound of the formula (I) according to claim 1, in which R is H and $R^1$ is H, $C_1$-$C_{18}$alkyl, $C_7$-$C_{18}$alkaryl, cyclohexyl, phenyl, naphthyl or a group of the formula (II)

$-(CH_2)_a-SR^3$ (II)

$R^2$ is $C_7$-$C_{18}$alkaryl, phenyl, naphthyl or phenyl containing, in the para-position, an HO group or a group of the formula

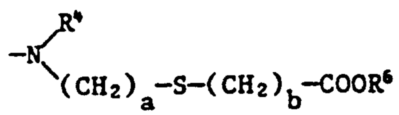

or

$R^1$ and $R^2$, together with the nitrogen atom to which they are attached, are a radical of the formula (III) according to claim 1, $R^3$ is a radical of the formula $-(CH_2)_b-COOR^6$, a is the number 1 or 3 and b is the number 1 or 2, $R^4$ is phenyl or a group of the formula $-(CH_2)_a-S-(CH_2)_b-COOR^6$ in which a and b are as defined above within claim 2, and $R^6$ is $C_1$-$C_{24}$alkyl, and, in the formula (III), the two $R^5$s are H, $C_1$-$C_{12}$alkyl or $C_9$phenylalkyl and X is -S-.

**3.** A compound of the formula (I) according to claim 1, in which R is H, $R^1$ is H, $CH_3$, $C_6H_5$, cyclohexyl, phenyl substituted by $C_4$-$C_{12}$alkyl, or a group of the formula $-CH_2-S-CH_2-COOR^6$, and $R^2$ is $C_6H_5$, naphthyl or phenyl substituted by $C_4$-$C_{12}$alkyl, or $R^1$ and $R^2$, together with the nitrogen atom to which they are attached, form a radical of the formula (III) according to claim 1, a is the number 1, $R^3$ is a radical of the formula $-CH_2COOR^6$ and $R^6$ is $C_4$-$C_{18}$alkyl, and, in the formula (III), the two $R^5$s are H or $C_4$-$C_8$alkyl and X is -S-.

**4.** A compound of the formula (I) according to claim 1, in which R is H, $R^1$ is H, $CH_3$, $C_6H_5$, cyclohexyl, phenyl containing an alkyl group having 8 C atoms, or a group of the formula $-CH_2-S-CH_2COOR^6$, and $R^2$ is $C_6H_5$, naphthyl or phenyl containing an alkyl group having 8 C atoms, or $R^1$ and $R^2$, together with the nitrogen atom to which they are attached, form a radical of the formula (III) according to claim 1, a is the number 1, $R^3$ is a radical of the formula $-CH_2COOR^6$ and $R^6$ is $C_8$-$C_{14}$alkyl or a mixture of $C_8H_{17}$ isomers, and, in the formula (III), the two $R^5$s are H or octyl and X is -S-.

**5.** A composition containing
a) at least one lubricant, one hydraulic fluid or one elastomer and
b) 0.01-10% by weight, relative to the lubricant, the hydraulic fluid or to the elastomer, of at least one compound of the formula (I) according to claim 1.

**6.** A composition according to claim 5, wherein the component a) is a lubricant.

**7.** A composition according to claim 5, wherein the component a) is an elastomer.

**8.** The use of the compounds of the formula (I) according to claim 1 as additives in lubricants, hydraulic fluids and elastomers.

**Claims for the following Contracting State : ES**

**1.** A composition containing (a) at least one lubricant, one hydraulic fluid or one elastomer and (b) 0.01-10% by weight, relative to the lubricant, the hydraulic fluid or to the elastomer, of at least one

compound of the formula (I)

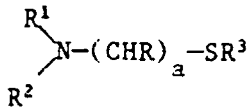

(I)

in which the Rs independently on one another are H, phenyl, naphthyl or $C_7$-$C_{30}$alkaryl and $R^1$ is H, $C_1$-$C_8$alkyl, $C_7$-$C_9$aralkyl, $C_5$-$C_{12}$cyclo-alkyl, phenyl, naphthyl, $C_7$-$C_{30}$alkaryl or a group of the formula (II)

$-(CH_2)_a-SR^3$ (II)

$R^2$ is $C_7$-$C_{30}$alkaryl, phenyl, naphthyl or phenyl containing an HO or $C_1$-$C_{18}$alkoxy group or a group of the formulae

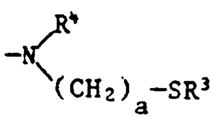 or 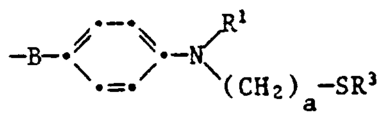 or 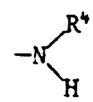

in the para-position, or $R^1$ and $R^2$, together with the nitrogen atom to which they are attached, are a radical of the formula (III)

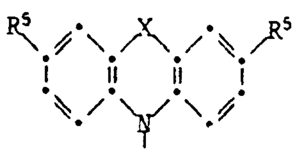

(III)

$R^3$ is a radical of the formulae $-(CH_2)_b-COOR^6$ or $-(CH_2)_2OCOR^7$, a is the number 1, 2 or 3 and b is the number 1 or 2, and $R^4$ is phenyl or a group of the formula (II), the two $R^5$s independently of one another are H, $C_1$-$C_{24}$alkyl, $C_5$-$C_{12}$cycloalkyl or $C_7$-$C_9$aralkyl, and $R^6$ is H, $C_1$-$C_{24}$alkyl or benzyl, $R^7$ is $C_1$-$C_{14}$alkyl, phenyl or a group of the formula

$-(CH_2)_m-Y-(CH_2)_m-COO(CH_2)_2-S-(CH_2)_aN(R^1)(R^2)$

in which Y is -O- or -S-, a is the number 1, 2 or 3 and m is the number 1 or 2, B is a direct bond, -S-, -S-S- or a $C_1$-$C_{12}$alkylene radical, X in the formula (III) is a direct bond, -S- or a group of the formulae

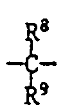 or 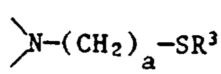

in which a and $R^3$ are as defined above, and $R^8$ and $R^9$ independently of one another are H, $C_1$-$C_8$alkyl or phenyl.

**2.** A composition according to claim 1, in which, in the compound of the formula (I), R is H and $R^1$ is H, $C_1$-$C_{18}$alkyl, $C_7$-$C_{18}$alkaryl, cyclohexyl, phenyl, naphthyl or a group of the formula (II)

$-(CH_2)_a-SR^3$ (II)

$R^2$ is $C_7$-$C_{18}$alkaryl, phenyl, naphthyl or phenyl containing, in the para-position, an HO group or a group of the formula

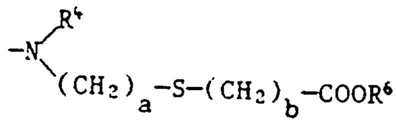

or

$R^1$ and $R^2$, together with the nitrogen atom to which they are attached, are a radical of the formula (III) according to claim 1, $R^3$ is a radical of the formula $-(CH_2)_b-COOR^6$, a is the number 1 or 3 and b is the number 1 or 2, $R^4$ is phenyl or a group of the formula $-(CH_2)_a-S(CH_2)_b-COOR^6$ in which a and b are as defined above within claim 2, and $R^6$ is $C_1$-$C_{24}$alkyl, and, in the formula (III), the two $R^5$s are H, $C_1$-$C_{12}$alkyl or $C_9$phenylalkyl and X is -S-.

3. A composition according to claim 1, in which, in the compound of the formula (I), R is H, $R^1$ is H, $CH_3$, $C_6H_5$, cyclohexyl, phenyl substituted by $C_4$-$C_{12}$alkyl, or a group of the formula $-CH_2-S-CH_2-COOR^6$, and $R^2$ is $C_6H_5$, naphthyl or phenyl substituted by $C_4$-$C_{12}$alkyl, or $R^1$ and $R^2$, together with the nitrogen atom to which they are attached, form a radical of the formula (III) according to claim 1, a is the number 1, $R^3$ is a radical of the formula $-CH_2COOR^6$ and $R^6$ is $C_4$-$C_{18}$alkyl, and, in the formula (III), the two $R^5$s are H or $C_4$-$C_8$alkyl and X is -S-.

4. A composition according to claim 1, in which, in the compound of the formula (I), R is H, $R^1$ is H, $CH_3$, $C_6H_5$, cyclohexyl, phenyl containing an alkyl group having 8 C atoms, or a group of the formula $-CH_2-S-CH_2COOR^6$, and $R^2$ is $C_6H_5$, naphthyl or phenyl containing an alkyl group having 8 C atoms, or $R^1$ and $R^2$, together with the nitrogen atom to which they are attached, form a radical of the formula (III) according to claim 1, a is the number 1, $R^3$ is a radical of the formula $-CH_2COOR^6$ and $R^6$ is $C_8$-$C_{14}$alkyl or a mixture of $C_8H_{17}$ isomers, and, in the formula (III), the two $R^5$s are H or octyl and X is -S-.

5. A composition according to claim 1, wherein the component a) is a lubricant.

6. A composition according to claim 1, wherein the component a) is an elastomer.

7. The use of the compounds of the formula (I) according to claim 1 as additives in lubricants, hydraulic fluids and elastomers.

8. A process for the preparation of a compound of the formula (I) defined in any one of claims 1 to 4, which comprises preparing a compound of the formula (I) in which R is H by reacting a compound of the formula (IV)

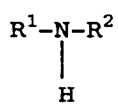

(IV)

in which $R^1$ and $R^2$ are as defined in the formula (I) in claim 1 with formaldehyde or a formaldehyde donor and with a compound of the formula (V)

$R^3SH$ (V)

in which $R^3$ is as defined in the formula (I), or preparing a compound of the formula (I) in which $R^1$ is H, a is the number 1 and R is phenyl, naphthyl or $C_7$-$C_{30}$alkaryl by first preparing an aldimine of the formula (VI)

$R^2-N=CH-R$ (VI)

by procedures known per se by reacting an amine of the formula $R^2NH_2$ with an aldehyde of the formula $O=CH-R$, and then reacting the aldimine thus formed with a compound of the formula $HSR^3$ to give the compound of the formula (I), $R^2$ and $R^3$ being as defined in claim 1, or preparing a compound of the formula (I) in which R is H and $R^3$ is a radical of the formula $-(CH_2)_2OCOR^7$ by reacting a

compound of the formula (VII)

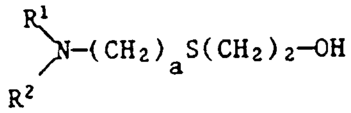

(VII)

by procedures known per se with an acid chloride of the formula $R^7$-COCl, an acid of the formula $R^7$-COOH or a methyl ester of an acid of the formula $R^7$-$COOCH_3$, in which $R^1$, $R^2$, $R^7$ and a are as defined in claim 1, or preparing a compound of the formula (I) in which a is the number 2 or 3 and R is H by a free-radical addition reaction, for example in the presence of catalysts or under irradiation, between a thio derivative of the formula (V) and a compound of the formula (VIII)

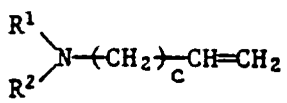

(VIII)

in which c is the number 0 or 1, $R^1$, $R^2$ and $R^3$ in the formulae (V) and (VIII) being as defined in claim 1, or preparing a compound of the formula (I) in which a is 2 or 3 and R is phenyl, naphthyl or $C_7$-$C_{30}$alkaryl by the same free-radical addition reaction described above.

**Revendications**
**Revendications pour les Etats contractants suivants : AT, BE, DE, FR, GB, IT, NL, SE**

**1.** Composés répondant à la formule (I) :

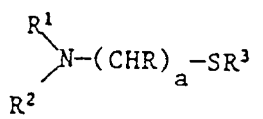

(I)

dans laquelle
les
R désignent indépendamment les uns des autres -H, un groupe phényle, naphtyle ou alkaryle en $C_7$-$C_{30}$, et
$R^1$ désigne -H, un groupe alkyle en $C_1$-$C_8$, aralkyle en $C_7$-$C_9$, cycloalkyle en $C_5$-$C_{12}$, phényle, naphtyle, alkaryle en $C_7$-$C_{30}$ ou un groupe répondant à la formule (II) :

$-(CH_2)_a-SR^3$ (II)

$R^2$ désigne un groupe alkaryle en $C_7$-$C_{30}$, phényle, naphtyle ou phényle qui présente en position para un groupe HO- ou alcoxy en $C_1$-$C_{18}$ ou un groupe répondant aux formules :

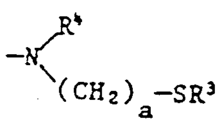

ou

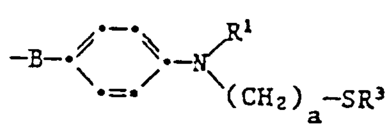

ou

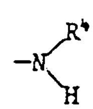

ou bien
$R^1$ et $R^2$ représentent avec l'atome d'azote auquel ils sont liés un radical répondant à la formule

(III) :

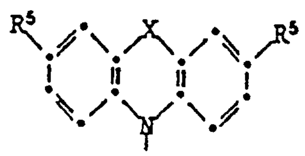

(III)

$R^3$ représente un radical répondant aux formules $-(CH_2)_b-COOR^6$ ou $-(CH_2)_2OCOR^7$,

a est un des nombres 1, 2 ou 3 et b un des nombres 1 ou 2, et

$R^4$ représente un groupe phényle ou un groupe répondant à la formule (II), les deux $R^5$ désignant indépendamment l'un de l'autre -H, un groupe alkyle en $C_1$-$C_{24}$, cycloalkyle en $C_5$-$C_{12}$ ou aralkyle en $C_7$-$C_9$, et

$R^6$ désigne -H, un groupe alkyle en $C_1$-$C_{24}$ ou benzyle,

$R^7$ désigne un groupe alkyle en $C_1$-$C_{14}$, phényle ou un groupe répondant à la formule :

$-(CH_2)_m-Y-(CH_2)_m-COO(CH_2)_2-S-(CH_2)_aN(R^1)(R^2)$

dans laquelle

Y représente -O- ou -S-, a les nombres 1, 2 ou 3 et m les nombres 1 ou 2.

B représente une liaison directe, -S-, -S-S- ou un radical alkylène en $C_1$-$C_{12}$,

X dans la formule (III) désigne une liaison directe, -S-ou un groupe répondant aux formules :

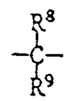

ou

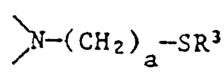

dans lesquelles a et $R^3$ ont les significations indiquées ci-dessus, et

$R^8$ et $R^9$ désignent indépendamment l'un de l'autre -H, un groupe alkyle en $C_1$-$C_8$ ou phényle,

**2.** Composés répondant à la formule (I) selon la revendication 1, dans lesquels :

R est -H, et

$R^1$ désigne -H, un groupe alkyle en $C_1$-$C_{18}$, alkaryle en $C_7$-$C_{18}$, cyclohexyle, phényle, naphtyle ou un groupe répondant à la formule (II) :

$-(CH_2)_a-SR^3$ (II)

$R^2$ désigne un groupe alkaryle en $C_7$-$C_{18}$, phényle, naphtyle ou phényle qui présente en position para un groupe HO- ou un groupe répondant à la formule :

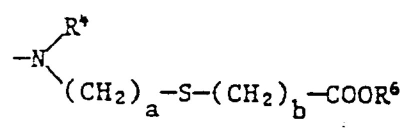

ou bien

$R^1$ et $R^2$ forment avec l'atome d'azote auquel ils sont liés un radical répondant à la formule (III) selon la revendication 1,

$R^3$ représente un radical répondant à la formule $-(CH_2)_b-COOR^6$,

a est un des nombres 1 ou 3 et b un des nombres 1 ou 2,

$R^4$ représente un groupe phényle ou un groupe répondant à la formule $-(CH_2)_a-S(CH_2)_b-COOR^6$, a et b ayant les significations indiquées dans la revendication 2, et

$R^6$ est un groupe alkyle en $C_1$-$C_{24}$, et, dans la formule (III), les deux radicaux
$R^5$ désignent -H, un groupe alkyle en $C_1$-$C_{12}$ ou phénylalkyle en $C_9$, et
X désigne -S-.

**3.** Composés répondant à la formule (I) selon la revendication 1, dans lesquels
R est -H,
$R^1$ est -H, $-CH_3$, $-C_6H_5$, un groupe cyclohexyle, un groupe phényle substitué par un groupe alkyle en $C_4$-$C_{12}$ ou un groupe répondant à la formule $-CH_2-S-CH_2-COOR^6$ et
$R^2$ désigne un groupe $-C_6H_5$, un groupe naphtyle ou un groupe phényle substitué par un groupe alkyle en $C_4$-$C_{12}$, ou bien
$R^1$ et $R^2$ forment avec l'atome d'azote auquel ils sont liés un radical répondant à la formule (III) selon la revendication 1,
a est le nombre 1,
$R^3$ est un radical répondant à la formule $-CH_2-COOR^6$, et
$R^6$ désigne un groupe alkyle en $C_4$-$C_{18}$ et, dans la formule (III), les deux radicaux
$R^5$ désignent -H ou un groupe alkyle en $C_4$-$C_8$, et
X désigne -S-.

**4.** Composés répondant à la formule (I) selon la revendication 1, dans lesquels
R est -H,
$R^1$ est -H, $-CH_3$, $-C_6H_5$, un groupe cyclohexyle, phényle contenant un groupe alkyle en $C_8$, ou un groupe répondant à la formule $-CH_2-S-CH_2-COOR^6$ et
$R^2$ est un groupe $-C_6H_5$, un groupe naphtyle ou un groupe phényle contenant un groupe alkyle en $C_8$, ou bien
$R^1$ et $R^2$ forment avec l'atome d'azote auquel ils sont liés un radical répondant à la formule (III) selon la revendication 1,
a est le nombre 1,
$R^3$ est un radical répondant à la formule $-CH_2-COOR^6$, et
$R^6$ désigne un groupe alkyle ou un mélange d'isomères en $C_8H_{17}$, et dans la formule (III), les deux radicaux
$R^5$ désignent -H ou un groupe octyle et
X désigne -S-.

**5.** Composition contenant
a) au moins un lubrifiant, un fluide hydraulique ou un élastomère et
b) 0,01-10 % en poids, par rapport au lubrifiant, au fluide hydraulique ou à l'élastomère, d'au moins un composé répondant à la formule (I) selon la revendication 1.

**6.** Composition selon la revendication 5, dans laquelle le constituant a) est un lubrifiant.

**7.** Composition selon la revendication 5, dans laquelle le constituant a) est un élastomère.

**8.** Utilisation des composés répondant à la formule (I) selon la revendication 1 comme additifs dans des lubrifiants, des fluides hydrauliques et des élastomères.

**Revendications pour l'Etat contractant suivant : ES**

**1.** Composition contenant
a) au moins un lubrifiant, un fluide hydraulique ou un élastomère et
b) 0,01-10 % en poids, par rapport au lubrifiant, au fluide hydraulique ou à l'élastomère, d'au moins un composé répondant à la formule (I) :

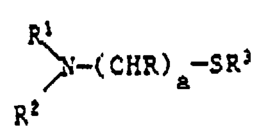

(I)

dans laquelle
les

R désignent indépendamment les uns des autres -H, un groupe phényle, naphtyle ou alkaryle en $C_7$-$C_{30}$, et

$R^1$ désigne -H, un groupe alkyle en $C_1$-$C_8$, aralkyle en $C_7$-$C_9$; cycloalkyle en $C_5$-$C_{12}$, phényle, naphtyle, alkaryle en $C_7$-$c_{30}$ ou un groupe répondant à la formule (II) :

$-(CH_2)_a-SR^3$ (II)

$R^2$ désigne un groupe alkaryle en $C_7$-$C_{30}$, phényle, naphtyle ou phényle, qui présente en position para un groupe HO- ou alcoxy en $C_1$-$C_{18}$ ou un groupe répondant aux formules :

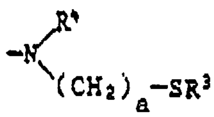 ou 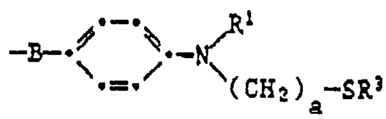 ou 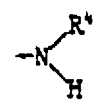

ou bien

$R^1$ et $R^2$ représentent avec l'atome d'azote auquel ils sont liés un radical répondant à la formule (III) :

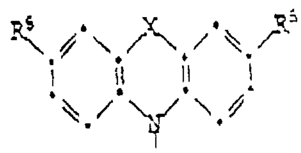 (III)

$R^3$ représente un radical répondant aux formules $-(CH_2)_b-COOR^6$ ou $-(CH_2)_2OCOR^7$,

a est un des nombres 1, 2 ou 3 et b un des nombres 1 ou 2, et

$R^4$ représente un groupe phényle ou un groupe répondant à la formule (II), les deux radicaux $R^5$ désignant indépendamment l'un de l'autre -H, un groupe alkyle en $C_1$-$C_{24}$, cycloalkyle en $C_5$-$C_{12}$ ou aralkyle en $C_7$-$C_9$, et

$R^6$ désigne -H, un groupe alkyle en $C_1$-$C_{24}$ ou benzyle,

$R^7$ désigne un groupe alkyle en $C_1$-$C_{14}$, phényle ou un groupe répondant à la formule :

$-(CH_2)_m-Y-(CH_2)_m-COO(CH_2)_2-S-(CH_2)_aN(R^1)(R^2)$

dans laquelle

Y représente -O- ou -S-, a les nombres 1, 2 ou 3 et m les nombres 1 ou 2.

B représente une liaison directe, -S-, -S-S- ou un radical alkylène en $C_1$-$C_{12}$,

X dans la formule (III) désigne une liaison directe, -S-ou un groupe répondant aux formules :

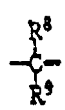 ou 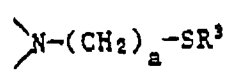

dans lesquelles a et $R^3$ ont les significations indiquées ci-dessus, et

$R^8$ et $R^9$ désignent indépendamment l'un de l'autre -H, un groupe alkyle en $C_1$-$C_8$ ou phényle,

**2.** Compositions selon la revendication 1, dans lesquelles, dans le composé répondant à la formule (I) :

R — est -H, et

$R^1$ — désigne -H, un groupe alkyle en $C_1$-$C_{18}$, alkaryle en $C_7$-$C_{18}$, cyclohexyle, phényle, naphtyle ou un groupe répondant à la formule (II) :

$$-(CH_2)_a-SR^3 \quad (II)$$

$R^2$ — désigne un groupe alkaryle en $C_7$-$C_{18}$ phényle, naphtyle ou phényle qui présente en position para un groupe HO- ou un groupe répondant à la formule :

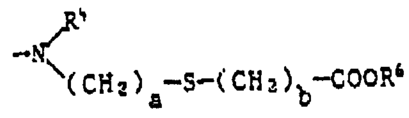

ou bien

$R^1$ et $R^2$ — forment avec l'atome d'azote auquel ils sont liés un radical répondant à la formule (III) selon la revendication 1,

$R^3$ — représente un radical répondant à la formule $-(CH_2)_b-COOR^6$,

a — est un des nombres 1 ou 3 et b un des nombres 1 ou 2,

$R^4$ — représente un groupe phényle ou un groupe répondant à la formule $-(CH_2)_a-S(CH_2)_b-COOR^6$, a et b ayant les significations indiquées ci-dessus dans la revendication 2, et

$R^6$ — est un groupe alkyle en $C_1$-$C_{24}$, et, dans la formule (III), les deux radicaux

$R^5$ — désignent -H, un groupe alkyle en $C_1$-$C_{12}$ ou phénylalkyle en $C_9$, et

X — désigne -S-.

**3.** Composition selon la revendication 1, dans laquelle, dans le composé répondant à la formule (I) :

R — est -H,

$R^1$ — est -H, $-CH_3$, $-C_6H_5$, un groupe cyclohexyle un groupe phényle substitué par un groupe alkyle en $C_4$-$C_{12}$ ou un groupe répondant à la formule $-CH_2-S-CH_2-COOR^6$, et

$R^2$ — désigne un groupe $-C_6H_5$, un groupe naphtyle ou un groupe phényle substitué par un groupe alkyle en $C_4$-$C_{12}$, ou bien

$R^1$ et $R^2$ — forment avec l'atome d'azote auquel ils sont liés un radical répondant à la formule (III) selon la revendication 1,

a — est le nombre 1,

$R^3$ — est un radical répondant à la formule $-CH_2-COOR^6$, et

$R^6$ — désigne un groupe alkyle en $C_4$-$C_{18}$ et, dans la formule (III), les deux radicaux

$R^5$ — désignent -H ou un groupe alkyle en $C_4$-$C_9$, et

X — désigne -S-,

**4.** Composition selon la revendication 1, dans laquelle, dans le composé répondant à la formule (I) :

R — est -H,

$R^1$ — est -H, $-CH_3$, $-C_6H_5$, un groupe cyclohexyle, phényle contenant un groupe alkyle en $C_8$, ou un groupe répondant à la formule $-CH_2-S-CH_2-COOR^6$ et

$R^2$ — est un groupe $-C_6H_5$, un groupe naphtyle ou un groupe phényle contenant un groupe alkyle en $C_8$, ou bien

$R^1$ et $R^2$ — forment avec l'atome d'azote auquel ils sont liés un radical répondant à la formule (III) selon la revendication 1,

a — est le nombre 1,

$R^3$ — est un radical répondant à la formule $-CH_2-COOR^6$, et

$R^6$ — désigne un groupe alkyle en $C_8$-$C_{14}$ ou un mélange d'isomères en $C_8H_{17}$, et, dans la formule (III), les deux radicaux

$R^5$ — désignant -H ou un groupe octyle et

X — désigne -S-.

**5.** Composition selon la revendication 1, dans laquelle le constituant a) est un lubrifiant.

**6.** Composition selon la revendication 1, dans laquelle le constituant a) est un élastomère.

**7.** Utilisation des composés répondant à la formule (I) selon la revendication 1 comme additifs dans des lubrifiants, des fluides hydrauliques et des élastomères.

**8.** Procédé de préparation de composés répondant à la formule (I) définis dans l'une des revendications 1 à 4, caractérisé an ce qu'on prépare des composés répondant à la formule (I) dans laquelle R est -H, en faisant réagir un composé répondant à la formule (IV) :

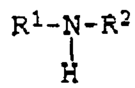

(IV)

dans laquelle $R^1$ et $R^2$ ont les significations indiquées dans la formule (I) de la revendication 1, avec du formaldéhyde ou un composé cédant du formaldéhyde et un composé répondant à la formule (V) :

$R^3$-SH (V)

dans laquelle $R^3$ a la signification indiquée dans la formule (I), ou bien on fait réagir les composés répondant à la formule (I), dans laquelle $R^1$ est -H, a est le nombre 1 et R désigne un groupe phényle, naphtyle ou alkaryle en $C_7$-$C_{30}$, en préparant d'abord une aldimine répondant à la formule (VI) :

$R^2$-N = CH-R (VI)

par réaction d'une amine répondant à la formule $R^2NH_2$ avec un aldéhyde répondant à la formule O = CH-R par un procédé connu en soi, puis on fait réagir l'aldimine ainsi formée avec un composé répondant à la formule $HSR^3$ pour donner un composé répondant à la formule (I), dans laquelle $R^2$ et$^3$ ont la signification indiquée dans la revendication 1, ou bien
on fait réagir les composés répondant à la formule (I), dans laquelle R est -H et $R^3$ représente un radical répondant a la formule $-(CH_2)_2OCOR^7$, en faisant réagir un composé répondant à la formule (VII) :

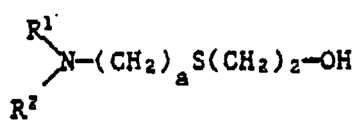

(VII)

avec un chlorure d'acide répondant à la formule $R^7$-COCl, un acide répondant à la formule $R^7$-COOH ou un ester méthylique d'acide répondant à la formule $R^7$-$COOCH_3$, par un procédé connu en soi, $R^1$, $R^2$, $R^7$ et a ayant les significations indiquées dans la revendication 1, ou bien
on prépare les composés répondant à la formule (I), dans laquelle a désigne les nombres 2 ou 3 et R est -H, en additionnant par voie radicalaire un dérivé thio répondant à la formule (V) sur un composé répondant à la formule (VIII) :

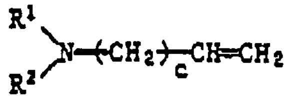

(VIII)

dans laquelle c désigne les nombres 0 ou 1, par exemple en présence de catalyseurs et sous irradiation, $R^1$, $R^2$ et $R^3$ ayant dans las formules (V) et (VIII) ayant la signification indiquée dans la revendication 1, ou bien
on prépare les composés répondant à la formule (I) dans laquelle a est 2 ou 3 et R un groupe phényle,

naphtyle ou alkaryle en $C_7$-$C_{30}$, par la même addition radicalaire que celle décrite ci-dessus.